# EUROPEAN PATENT APPLICATION

(11) **EP 2 944 319 A1**
(43) Date of publication of application: **18.11.2015**
(21) Application number: 15167715.0
(22) Date of filing: 08.11.2011
(51) Int. Cl.: A61K 38/55

(54) **BUFFERED OPHTHALMIC COMPOSITIONS AND USE THEREOF**

(30) Priority: 08.11.2010 US 411466 P; 08.11.2010 US 411464 P
(62) Divisional of application: 11840613.1
(71) Applicant: Arava Bio-Tech Ltd., 42902 Netanya (IL)
(72) Inventor: Tennenbaum, Tamar, 9610510 Jerusalem (IL); Braiman-Wiksman, Liora, 7530863 Rishon LeZion (IL); Sagiv, Yuval, 7041810 Gedera (IL); Levy-Hacham, Ofra, 74140 Ness Ziona (IL)
(74) Representative: Pearl Cohen Zedek Latzer Baratz UK LLP

(57) **Abstract**

Use of a PKC-α inhibitor or a physiologically acceptable salt thereof in the preparation of an ophthalmic composition for treating dry eye in a subject.

## Description

### BACKGROUND OF THE DISCLOSURE

### FIELD OF THE DISCLOSURE

The disclosure relates generally to ophthalmic compositions and more specifically to ophthalmic buffers for formulation of topically acceptable suspensions useful in delivery of pharmaceutically active agents and treatment of eye disorders, injuries and diseases. The disclosure also relates to methods of treating eye disorders, injuries and diseases with the ophthalmic buffer formulations of the invention, alone or in combination with pharmaceutically active agents, and for methods of making the buffers and formulations based on the buffers. The buffered solutions work especially well with PKC activators or inhibitors, e.g., PKCα inhibitors.

### BACKGROUND INFORMATION

A variety of eye disorders resulting from disease or injury have been described and are treated with varying success rates. For example, one disorder is a corneal ulcer, which is an open sore on the surface cornea occurring as a result of bacteria, viral or fungi infection, mechanical injury or severe allergic disease. A corneal ulcer is a serious condition that must be treated promptly to avoid lasting vision problems. Inflammation of the cornea due to infection or injury can cause severe loss of vision and even blindness. The deeper the cornea ulcer, the more serious the condition becomes and very deep ulcers can result in scarring on the cornea subsequently blocking light from entering the eye. Treatment usually involves antibiotics as well as antiviral or antifungal medications. Steroid eye drops may also be given to reduce inflammation. However, there are no drugs that enhance wound closure and prevent scarring. In severe cases when the existing therapies are not helpful a corneal transplant may be needed to restore vision.

Chemical injuries to the eye represent one of the true ophthalmic emergencies which results from either strongly basic (alkaline) compounds or acidic compounds. Alkali injuries are more common and can be more deleterious especially in bilateral chemical exposure that often results in permanent visual damage. The severity of this injury is related to type, volume, concentration, duration of exposure, and degree of penetration of the chemical. 20% of chemical injuries result in significant visual and cosmetic disability; only 15% of patients with severe chemical injuries achieve functional visual rehabilitation. Corneal epithelial damage can range from mild diffuse punctate epithelial keratitis (PEK) to a complete epithelial defect. These wounds are also characterized by exuberant conjunctival inflammation and anterior chamber inflammatory reaction as well as corneal perforation.

Dry eye is considered a multifactorial disease of the tears and the ocular surface that results in symptoms of discomfort, visual disturbance, and tear film instability with potential damage to the ocular surface. Dry eye is usually accompanied by increased osmolarity of the tear film and inflammation of the ocular surface.

Aqueous tear deficiency (ATD) is usually the most common cause of dry eye, and it may be due to insufficient tear production. The secretion of the lacrimal gland is controlled by a neural reflex arc, with afferent nerves (trigeminal sensory fibers) in the cornea and the conjunctiva passing to the pons (superior salivary nucleus), from which efferent fibers pass, in the nervus intermedius, to the pterygopalatine ganglion and postganglionic sympathetic and parasympathetic nerves terminating in the lacrimal glands. Keratoconjunctivitis sicca (KCS) is the name given to this ocular surface disorder. KCS is usually subdivided into Sjögren syndrome (SS) associated KCS and non-SS associated KCS. Patients with aqueous tear deficiency have SS if they have associated xerostomia and/or connective tissue disease. Patients with primary SS have evidence of a systemic autoimmune disease as manifested by the presence of serum autoantibodies and very severe aqueous tear deficiency and ocular surface disease. Non-SS KCS is mostly due to decrease in androgens, such as either from reduced ovarian function in the postmenopausal female or from increased levels of the sex hormone binding globulin in pregnancy and birth control pill use.

Dry eye may be complicated by sterile or infectious corneal ulceration, particularly in patients with SS. Ulcers are typically oval or circular, less than 3 mm in diameter, and located in the central or paracentral cornea. Occasionally, corneal perforation may occur. In rare cases, sterile or infectious corneal ulceration in dry eye syndrome can cause blindness. Other complications may include punctate epithelial defects (PEDs), corneal neovascularization, and corneal scarring.

Treatment of eye disorders typically requires delivery of pharmaceutically active agents to the eye, such as with or via buffered liquid ophthalmic solutions. Alternatively, some treatments require specifically buffered solutions that provide lubrication to the eye to ameliorate disorders. Ideally ophthalmic solutions must be formulated to accommodate pharmaceutically active agents as well as being compatible with the physiology of the eye.. As such, it would be useful to have ophthalmic buffered solutions that provide treatment as well as accommodate pharmaceutically active agents, and preferably work synergistically by enhancing the mechanism of action of the agent to increase the rate of healing that is provided by either the ophthalmic solution or active agent alone.

### SUMMARY

The present disclosure is based in part on the discovery of ophthalmic buffers for formulation of topically administrable suspensions useful in delivery of pharmaceutically active agents as well as treating eye disorders by accelerating or promoting healing of damaged ocular tissue.

Accordingly, in one aspect, the present disclosure provides a liquid ophthalmic composition. The composition includes: a) 0.1 to 2.0% (w/v) sodium chloride; b) 0.01 to 0.5% (w/v) potassium chloride; c) 0.01 to 1.0% (w/v) sodium acetate trihydrate; d) 0.01 to 1.0% (w/v) trisodium citrate dihydrate; and e) water. In one embodiment, the composition does not include, or includes less than 0.03% (w/v) of both, or either of calcium chloride dihydrate or magnesium chloride hexahydrate.

In another embodiment, the present disclosure provides a liquid ophthalmic composition including: a) 0.1 to 2.0% (w/v) sodium chloride; b) 0.01 to 0.5% (w/v) potassium chloride; c) 0.01 to 1.0% (w/v) sodium acetate trihydrate; d) 0.01 to 1.0% (w/v) trisodium citrate dihydrate; e) water; and f) a pharmaceutically active agent. In some embodiments the pharmaceutically active agent is a PKC inhibitor or activator, such as a PKC-α inhibitor, a PKC-ε inhibitor, a PKC-δ inhibitor or a PKC-δ activator. In some embodiments the pharmaceutically active agent is a PKC-α inhibitor. In some embodiments, the composition does not include, or includes less than 0.03% (w/v) of both, or either of calcium chloride dihydrate or magnesium chloride hexahydrate.

In another aspect, the present disclosure provides a method of accelerating or promoting healing of damaged ocular tissue or an ocular wound in a subject. The method includes administering a liquid ophthalmic composition as disclosed to an eye of a subject. In various embodiments, the wound may be a corneal ulceration wound, a retinopathy wound, a burn, an inflammation wound, a dry eye syndrome wound, a macular degeneration wound, a laceration, a surgical incision wound, or a post surgical adhesion wound.

In another aspect, the present disclosure provides a method of lubricating an eye of a subject. The method includes topically administering a liquid ophthalmic composition to an eye of the subject in an amount sufficient to lubricate the eye. In a preferred embodiment, the ophthalmic composition is adapted for use as an ocular lubricant or artificial tear composition.

In another aspect, the present disclosure provides a method of treating an infection an eye of a subject. The infection may be caused by any infectious agent, for instance, viral, fungal or bacterial, protozoal, amoebic, or the like. The method includes topically administering a liquid ophthalmic composition to an eye of the subject. In a preferred embodiment, the ophthalmic composition of the invention containing an anti-infective agent or an anti-inflammatory agent in amounts sufficient to treat or cure the infection.

Another aspect of the invention relates to treating allergies of the eye by administering a composition based on the buffered ophthalmic solutions of the present invention that additionally contain an anti-allergic medication such as an antihistamine, e.g., a corticosteroid, a mast cell stabilizer such as cromolyn sodium, a vasoconstrictor such as naphazoline.

The present invention also relates to formulations to treat glaucoma that are based on the buffers of the present invention, and for methods of treating glaucoma with such compositions. Typically, these formulations will contain anti-glaucoma agents such as β-blockers, adrenergic agonists, for example, α-3 adrenergic agonists, demecarium bromide, or any other known or unyet discovered agent which can treat glaucoma by topical delivery to the eye.

In another aspect, the ophthalmic compositions of the present disclosure are useful as an eye wash or flush to prevent or inhibit ocular tissue injury or wounding. As such, the present disclosure provides method of preventing or inhibiting ocular tissue injury or wounding including topically administering a liquid ophthalmic composition of the present disclosure to an eye of the subject in response to exposure to a caustic chemical.

In another aspect, the present disclosure provides a method of delivering a pharmaceutical agent to a subject having a disease, condition or injury to the eye. The method includes topically administering a liquid ophthalmic composition of the present disclosure to an eye of the subject, wherein the composition includes one or more pharmaceutically active agents and is administered in an amount sufficient to treat or cure the said injury, disease or condition.

In various embodiments, the liquid ophthalmic composition includes: 0.6 to 0.8% (w/v) sodium chloride; 0.07 to 0.09% (w/v) potassium chloride; 0.3 to 0.5% (w/v) sodium acetate trihydrate; 0.1 to 0.3% (w/v) trisodium citrate dihydrate; and sterile water. In some embodiments, the composition has a pH of about 5.5 to 8.0 or 6.8 to 7.6 and an osmolality from about 220 to 320 mOsm/kg. In various embodiments, the composition has a viscosity of about 1 to 50,000 cps or about 1 to 4,000 cps.

As eluded to above, compositions based on the buffered solutions of the present invention may include one or more pharmaceutically active agents which may be used to topically treat or cure a condition, disease or injury of the eye, including, but not limited to, anesthetics, astringents, anti-hypertensives, anti-glaucoma agents, neuro-protective agents, anti-allergy agents, muco-secretagogues, angiostatics, anti-microbials, pain-relieving or anti-inflammatory agents.

In certain embodiments, the pharmaceutically active agent is preferably a polypeptide, oligonucleotide, hormone, steroid, corticosteroid, chemical compound, or lipid. In some embodiments the pharmaceutically active agent is a PKC inhibitor or activator, such as a PKC-α inhibitor, a PKC-ε inhibitor, a PKC-δ inhibitor or a PKC-δ activator. In some embodiments the PKC inhibitor or activator is a polypeptide selected from SEQ ID NOs: 1-18.

### BRIEF DESCRIPTION OF THE DRAWINGS

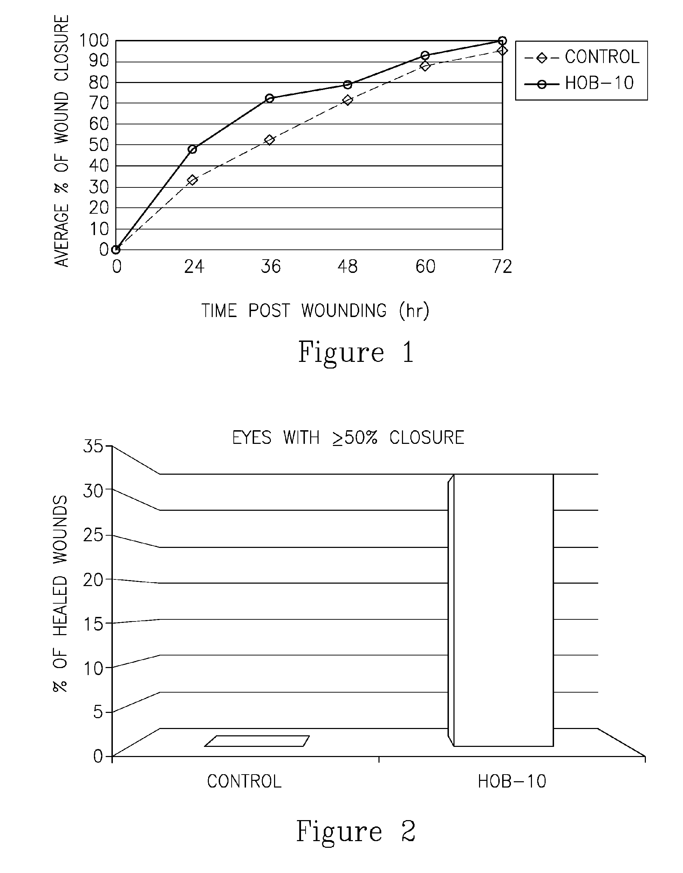

Figure 1 is a graphical representation comparing the therapeutic effect of ophthalmic buffer solution Formula 1 with Control (DPBS^{-/-}) in rabbit eyes subject to mechanical corneal wounding.
Figure 2 is a graphical representation comparing the therapeutic effect of ophthalmic buffer solution Formula 1 with Control (BSS) in rabbit eyes subject to mechanical corneal wounding. The graph depicts the percentage of eyes with ≥ 50% reduction in wound size within 24 hr post wounding.

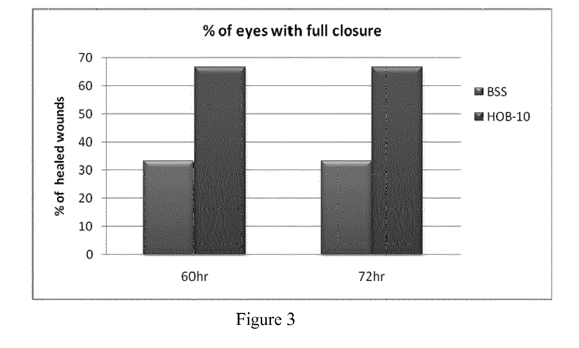

Figure 3 is a graphical representation comparing the therapeutic effect of ophthalmic buffer solution Formula 1 with Control (BSS) in rabbit eyes subject to mechanical corneal wounding. The graph depicts the percentage of eyes with complete wound closure within 60 hr and 72 hr post wounding.

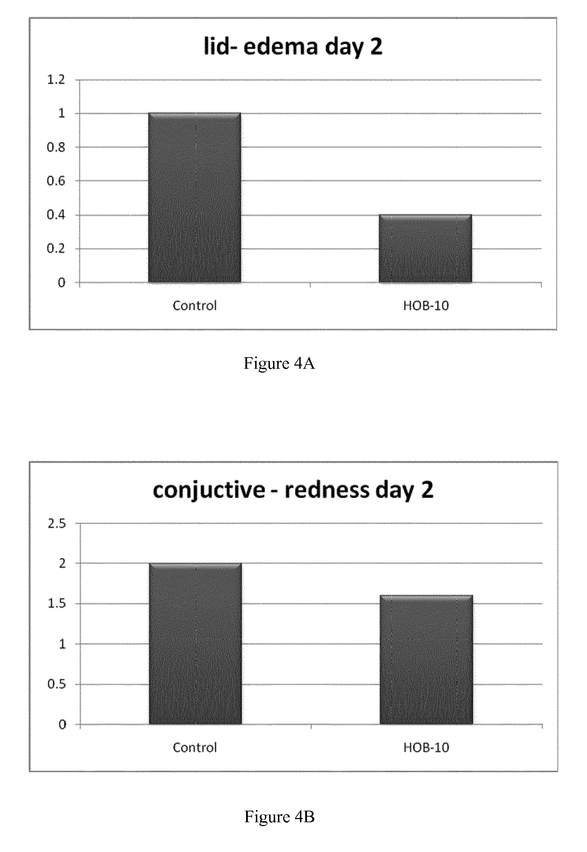

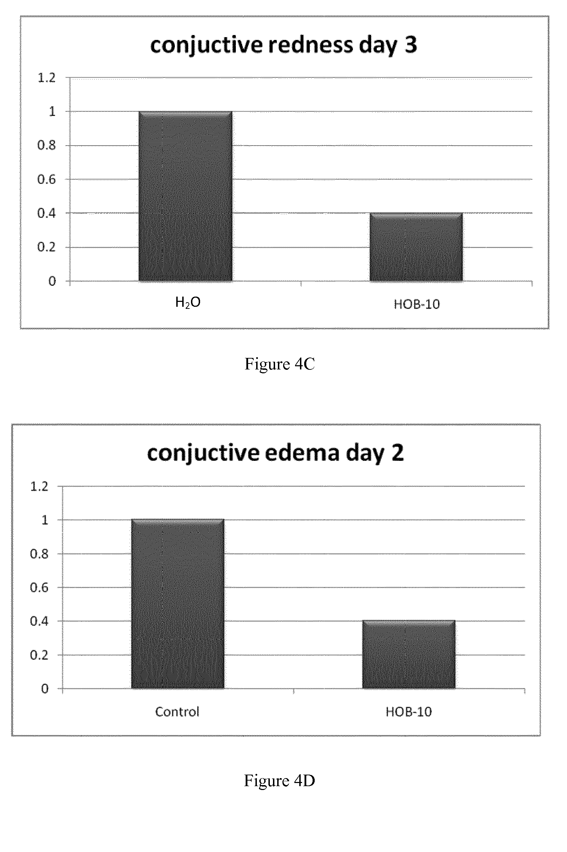

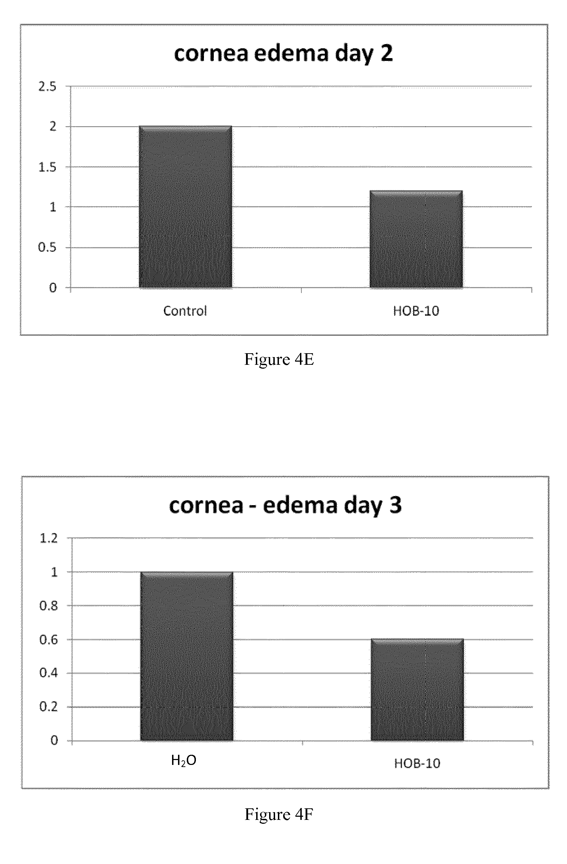

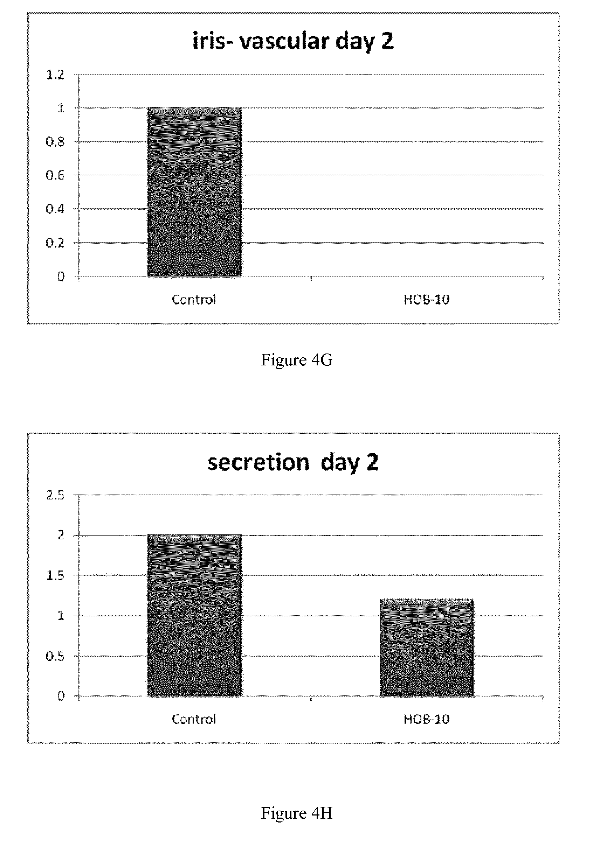

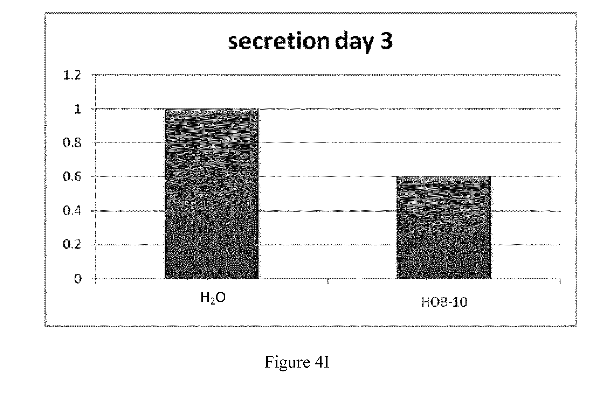

Figures 4A through 4I are a series of graphical representations comparing the therapeutic effect of ophthalmic buffer solution Formula 1 with Control in rabbit eyes subject to chemical corneal wounding. Figure 4A compares lid edema 2 days post wounding. Figure 4B compares conjunctive redness 2 days post wounding. Figure 4C compares conjunctive redness 3 days post wounding. Figure 4D compares conjunctive edema 2 days post wounding. Figure 4E compares cornea edema 2 days post wounding. Figure 4F compares cornea edema 3 days post wounding. Figure 4G compares iris vascularization 2 days post wounding. Figure 4H compares eye secretion 2 days post wounding. Figure 4I compares eye secretion 3 days post wounding.

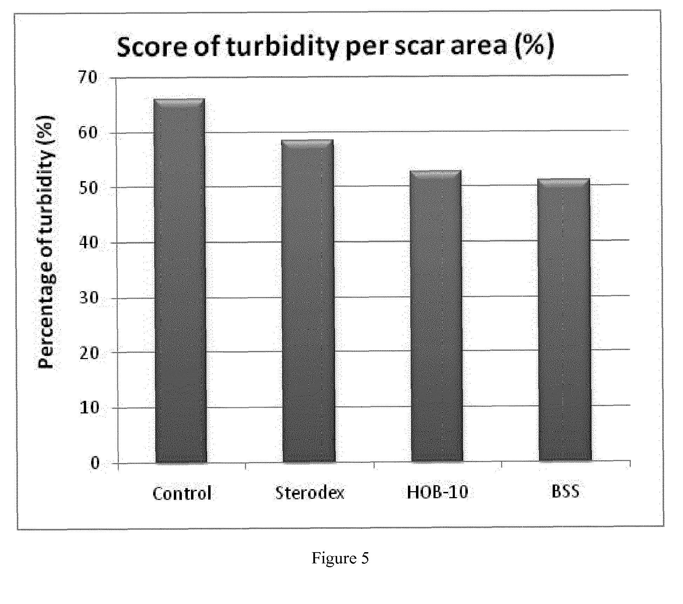

Figure 5 is a graphical representation comparing the therapeutic effect of ophthalmic buffer solution Formula 1 with BSS, Sterodex® (standard of care) and Control (water) in rabbit eyes subject to chemical corneal wounding. The graph depicts the score of turbidity per scar area after 7 days of treatment.

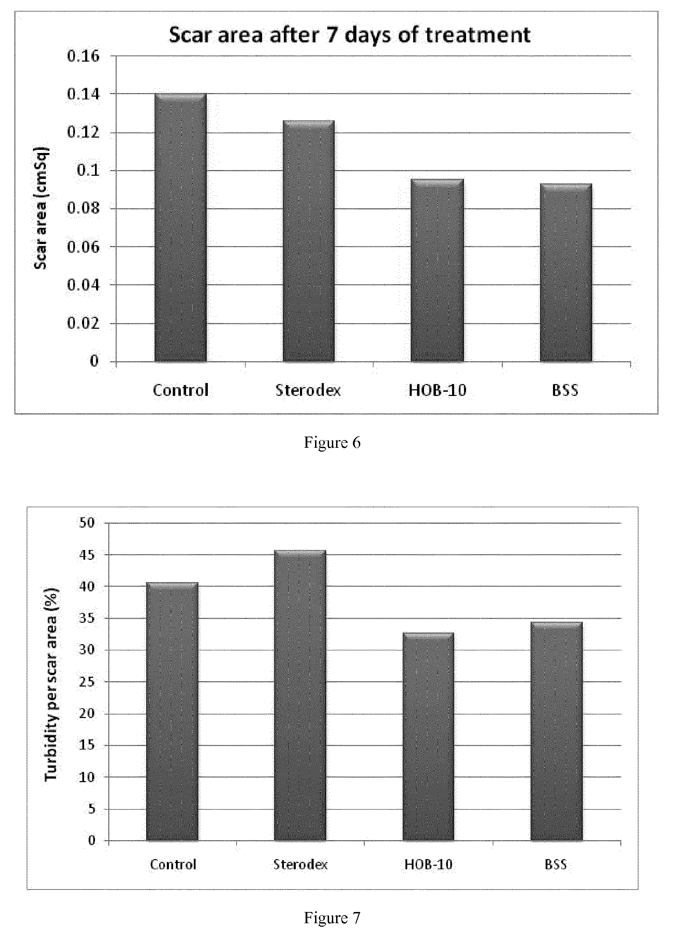

Figure 6 is a graphical representation comparing the therapeutic effect of ophthalmic buffer solution Formula 1 with BSS, Sterodex® (standard of care) and Control (water) in rabbit eyes subject to chemical corneal wounding. The graph depicts the size of scar area after 7 days of treatment.
Figure 7 is a graphical representation comparing the therapeutic effect of ophthalmic buffer solution Formula 1 with BSS, Sterodex® (standard of care) and Control (water) in rabbit eyes subject to chemical corneal wounding. The graph depicts the score of turbidity per scar area after 7 days of treatment.

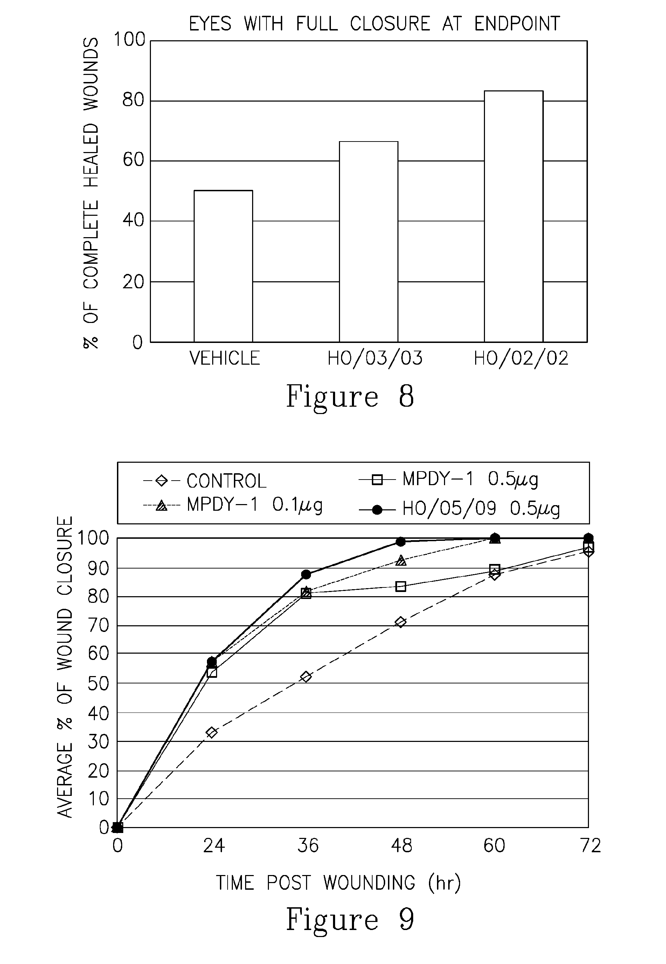

Figure 8 is a graphical representation including a histogram comparing the percent of completely healed corneas subjected to mechanical corneal wounding and treated with vehicle alone (DPBS^{-/-}, left), Formula 3 (MPDY-1 0.1µg/eye/treatment and insulin 0.01U/eye/treatme (center), and Formula 2 (MPDY-1 0.1µg/eye/treatment, right).
Figure 9 is a graphical representation comparing the therapeutic effect of the polypeptide MPDY-1 in BSS (standard ophthalmic buffer) or in HOB-10 (referred to as HO/05/09). Concentrations of MPDY-1 are shown in µg/eye/treatment.

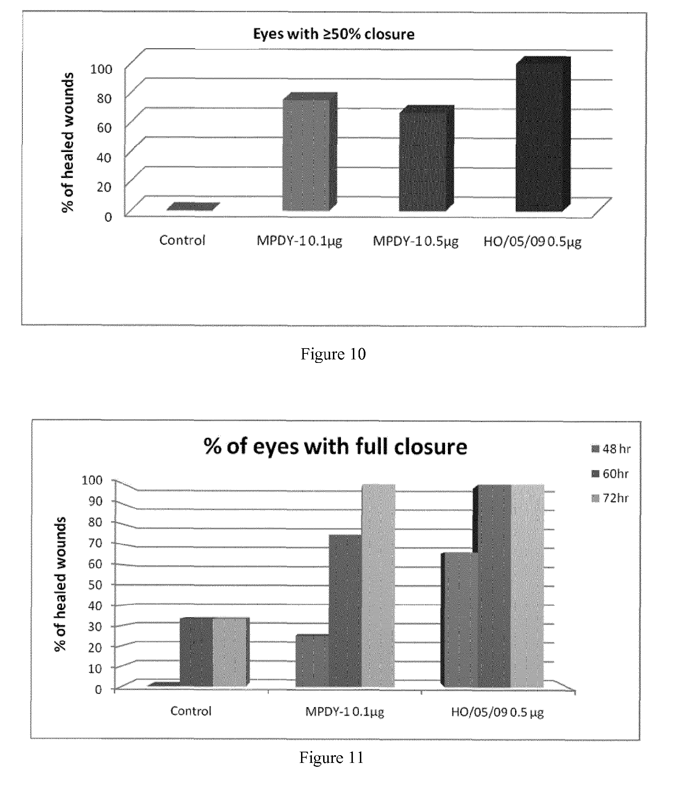

Figure 10 is a graphical representation including a histogram of the comparing the percentage of mechanically injured eyes with ≥ 50% closure at 24 hours after treatment with the polypeptide MPDY-1 in BSS (standard ophthalmic buffer) or in HOB-10 (referred to as HO/05/09). Concentrations of MPDY-1 are shown in µg/eye/treatment.
Figure 11 is a graphical representation including a histogram of the comparing the percentage of eyes with full closure at 48, 60 and 72 hours after treatment with the polypeptide MPDY-1 in BSS (standard ophthalmic buffer) or in HOB-10 (referred to as HO/05/09). Concentrations of MPDY-1 are shown in µg/eye/treatment.

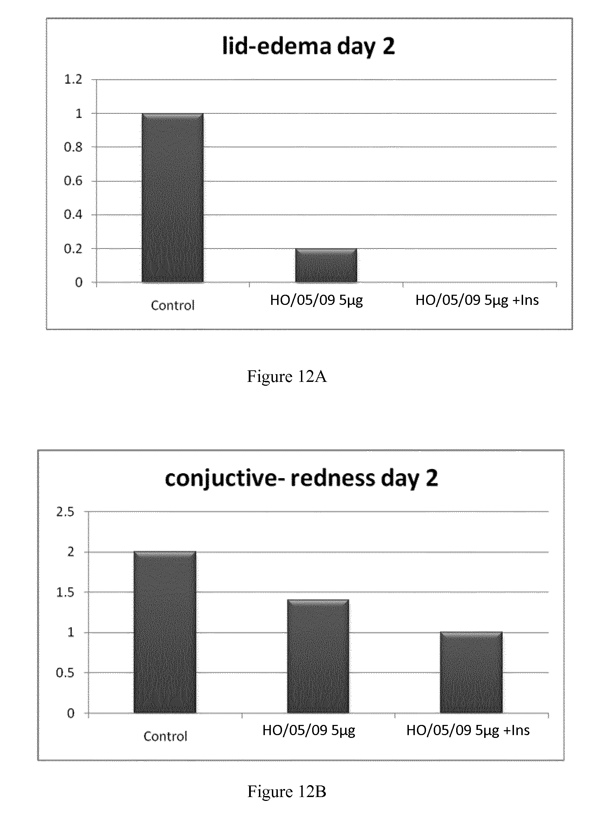

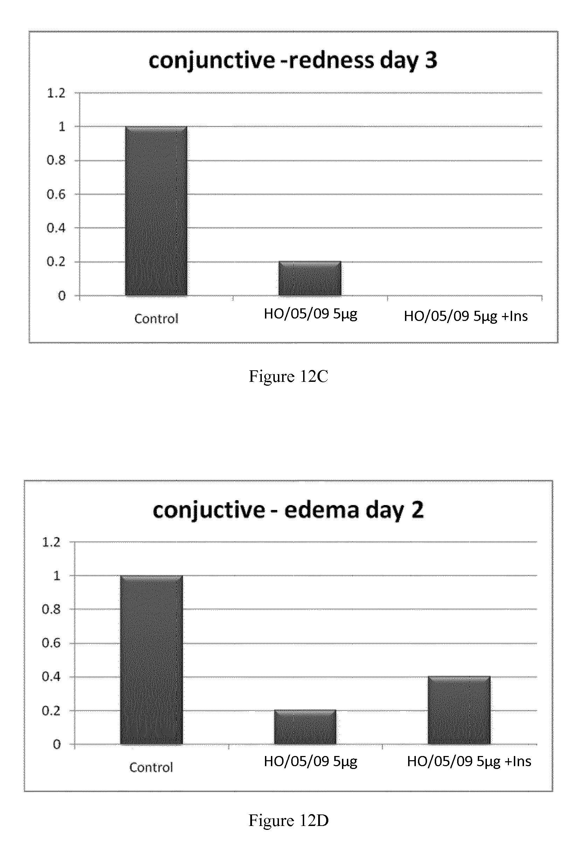

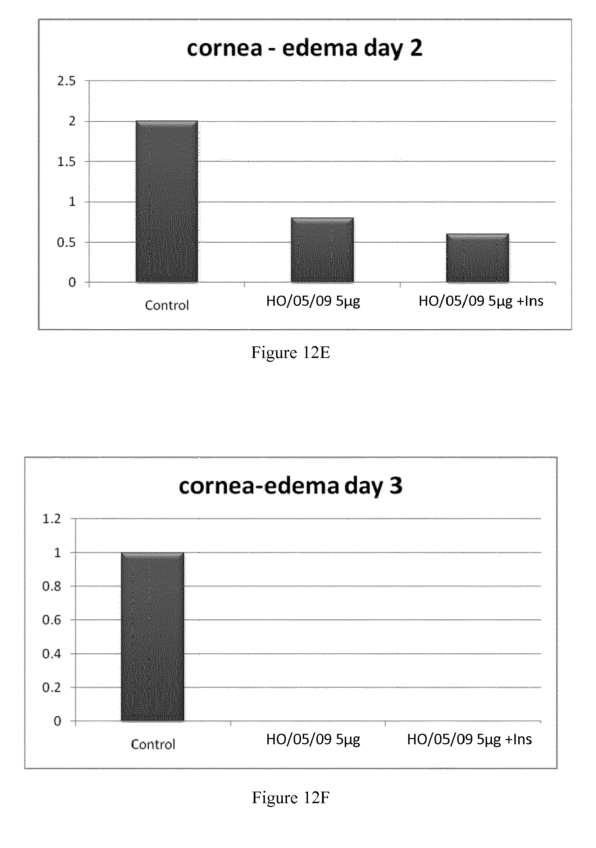

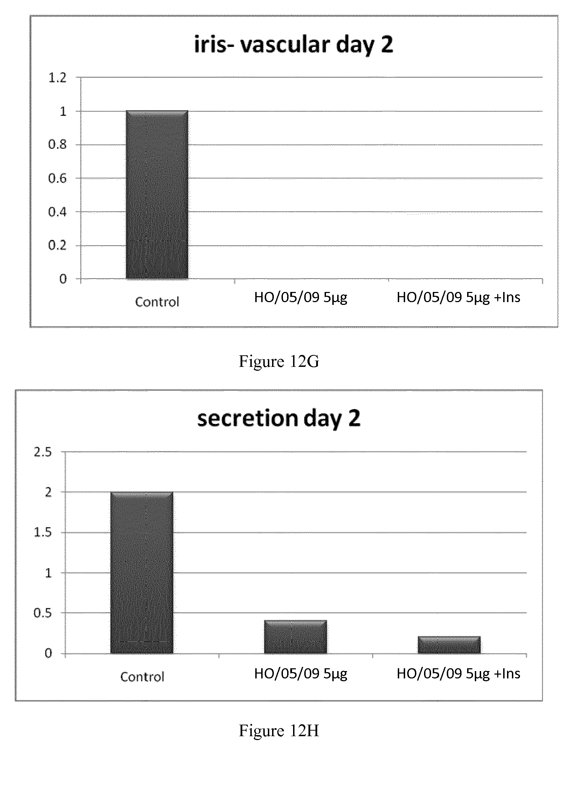

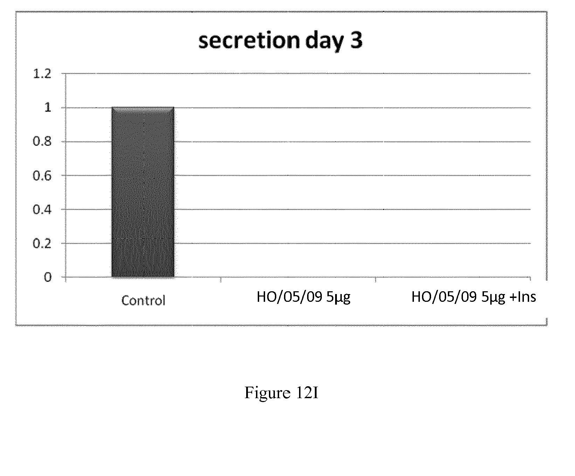

Figures 12A through 12I are series graphical representations comparing the therapeutic effect of the polypeptide MPDY-1 alone (HO/05/09) and in combination with insulin(HO/05/09 + Ins), with Control in rabbit eyes subject to chemical corneal wounding. Figure 12A compares lid edema 2 days post wounding. Figure 12B compares conjunctive redness 2 days post wounding. Figure 12C compares conjunctive redness 3 days post wounding. Figure 12D compares conjunctive edema 2 days post wounding. Figure 12E compares cornea edema 2 days post wounding. Figure 12F compares cornea edema 3 days post wounding. Figure 12G compares iris vascularization 2 days post wounding. Figure 12H compares eye secretion 2 days post wounding. Figure 12I compares eye secretion 3 days post wounding.

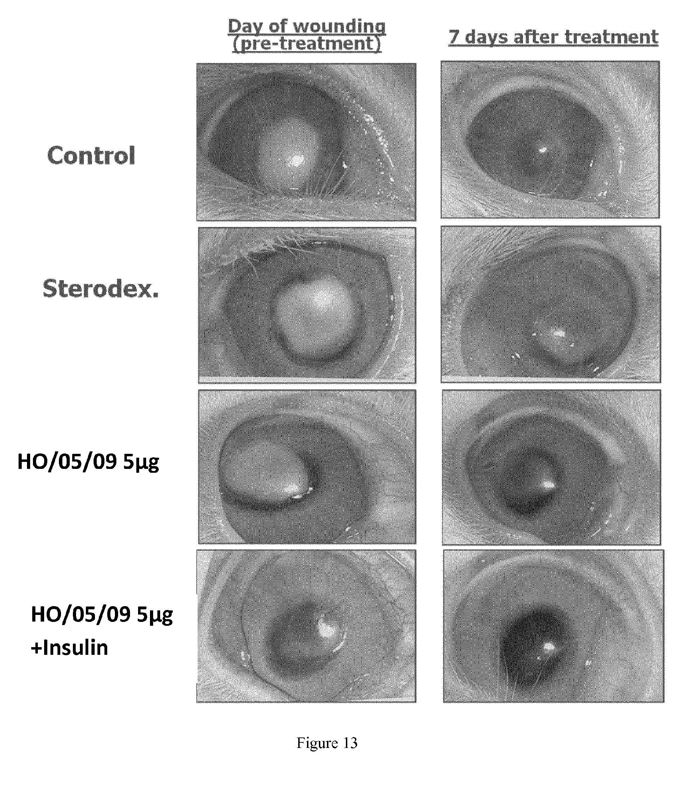

Figure 13 is a pictorial representation including images of rabbit eye subject to chemical corneal wounding and treated with polypeptide MPDY-1 alone in HOB-10 (referred to as HO/05/09), and in combination with insulin (referred to as HO/05/09 + Insulin), with Sterodex® (standard of care) and Control (water).

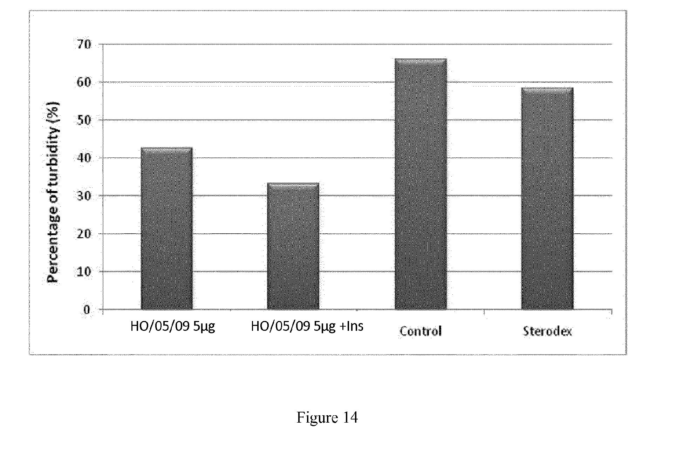

Figure 14 is a graphical representation comparing the therapeutic effect of the polypeptide MPDY-1 alone in HOB-10 (referred to as HO/05/09), and in combination with insulin (referred to as HO/05/09 + Ins), with Sterodex® (standard of care) and Control (water) in rabbit eyes subject to chemical corneal wounding. The graph depicts the score of turbidity per scar area after 7 days of treatment.

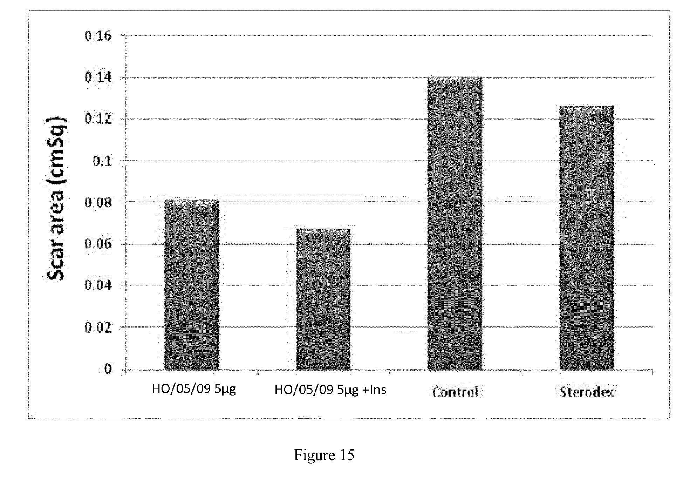

Figure 15 is a graphical representation comparing the therapeutic effect of the polypeptide MPDY-1 alone in HOB-10 (referred to as HO/05/09), and in combination with insulin (referred to as HO/05/09 + Ins), with Sterodex® (standard of care) and Control (water) in rabbit eyes subject to chemical corneal wounding. The graph depicts the size of scar area after 7 days of treatment.

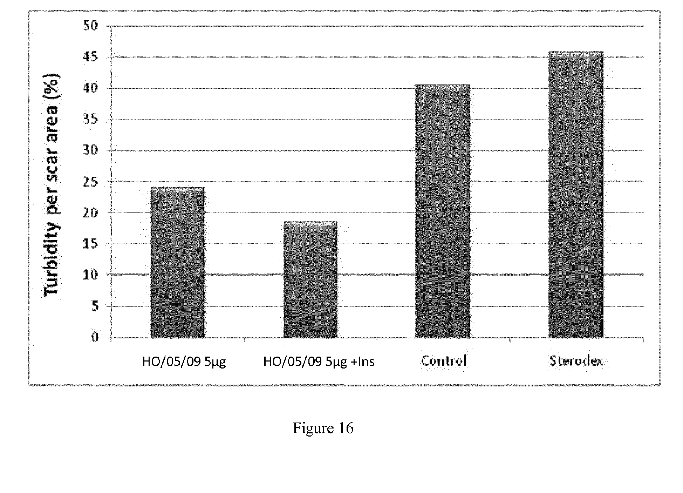

Figure 16 is a graphical representation comparing the therapeutic effect of the polypeptide MPDY-1 alone in HOB-10 (referred to as HO/05/09), and in combination with insulin (referred to as HO/05/09 + Ins), with Sterodex® (standard of care) and Control (water) in rabbit eyes subject to chemical corneal wounding. The graph depicts the score of turbidity per scar area after 7 days of treatment.

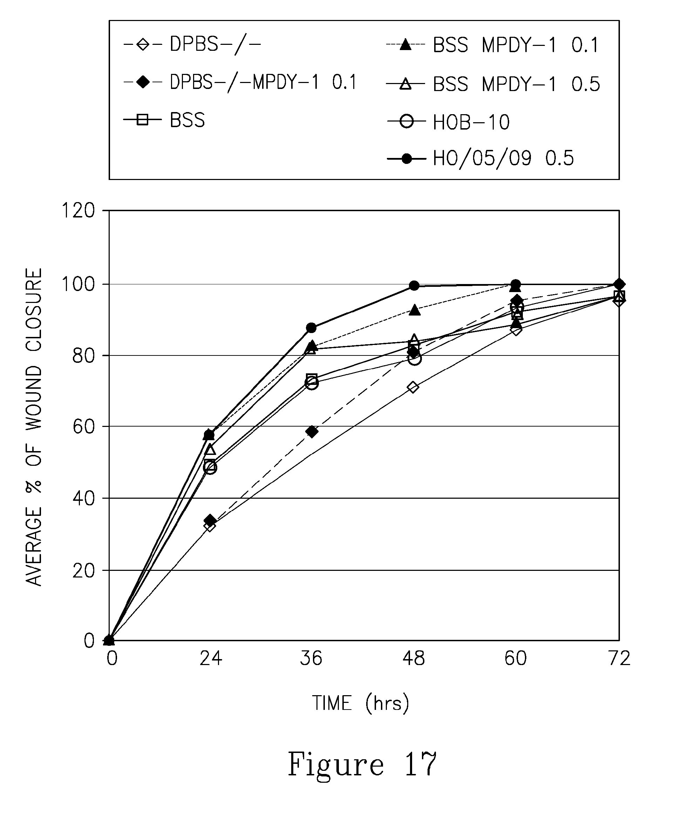

Figure 17 is a graphical representation comparing the therapeutic effect of the polypeptide MPDY-1 in combination with different ophthalmic buffers. Concentrations of MPDY-1 (µg/eye/treatment) are shown.

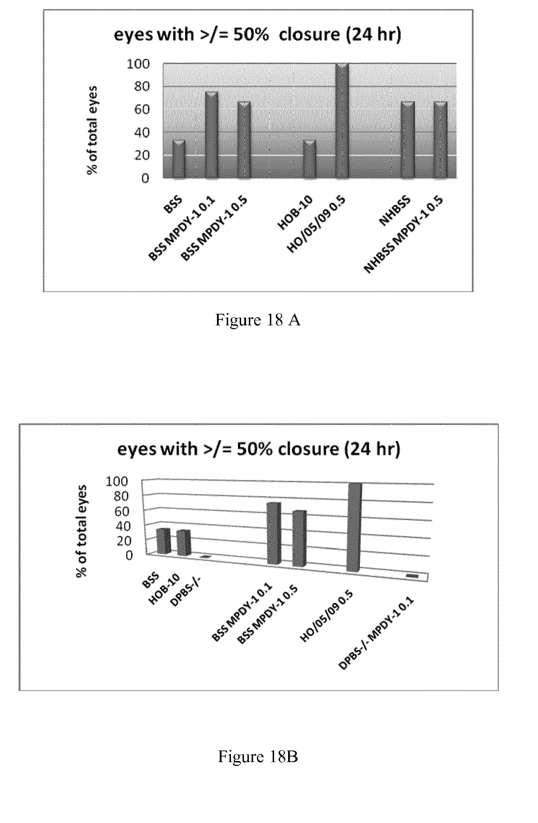

Figures 18A and 18B are graphical representation including histograms of the percentage of eyes with ≥ 50% closure at 24 hours after treatment with the polypeptide MPDY-1 in combination with different ophthalmic buffers. Concentrations of MPDY-1 (µg/eye/treatment) are shown.

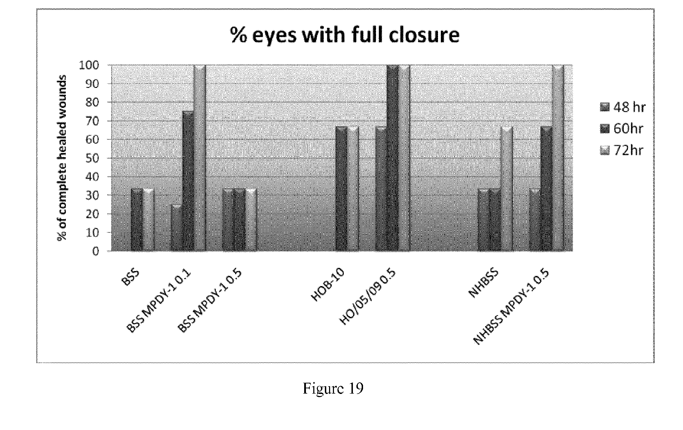

Figure 19 is a graphical representation including histograms of the percentage of eyes with full closure at 48, 60 and 72 hours after treatment with the polypeptide MPDY-1 in combination with different ophthalmic buffers.

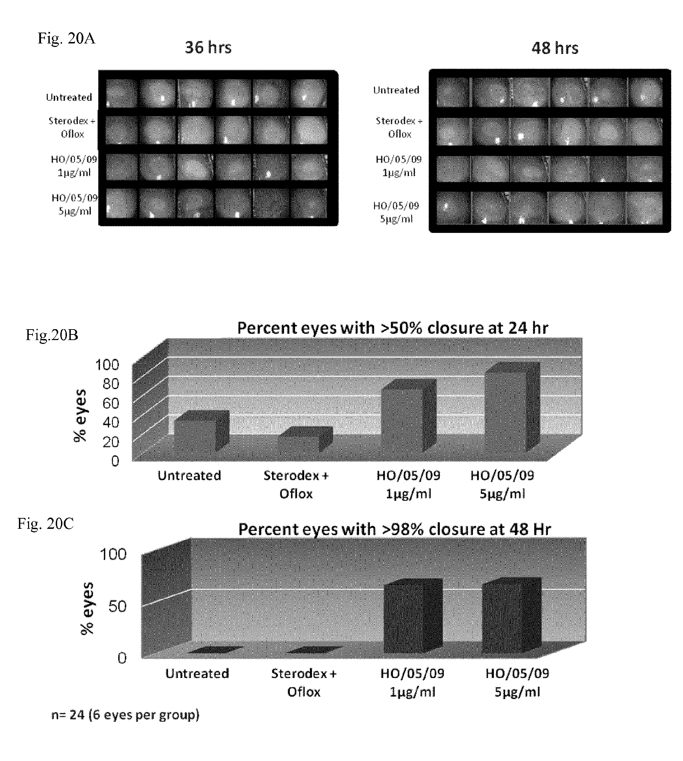

Figure 20 is a series of pictorial and graphical representations showing promotion of re-epithelialization of corneal wounding by HO/05/09. Rabbits were subjected to chemical alkali burn wounding (6mm wide, NaOH IN for 20 seconds). Eyes were treated by three daily ocular instillations of treatments during 7 successive days. Fluorescein staining was used to measure the corneal erosion immediately after wounding, at 24, 36, 48 and 60 hours post wounding and at the end of study. Figure 20A depicts fluorescein-stained corneal images. Figure 20B depicts the percentage of wounds that achieved 50% closure at 24 hours post wounding. Figure 20C depicts the percentage of wounds that achieved 98% closure at 48 hours post wounding.

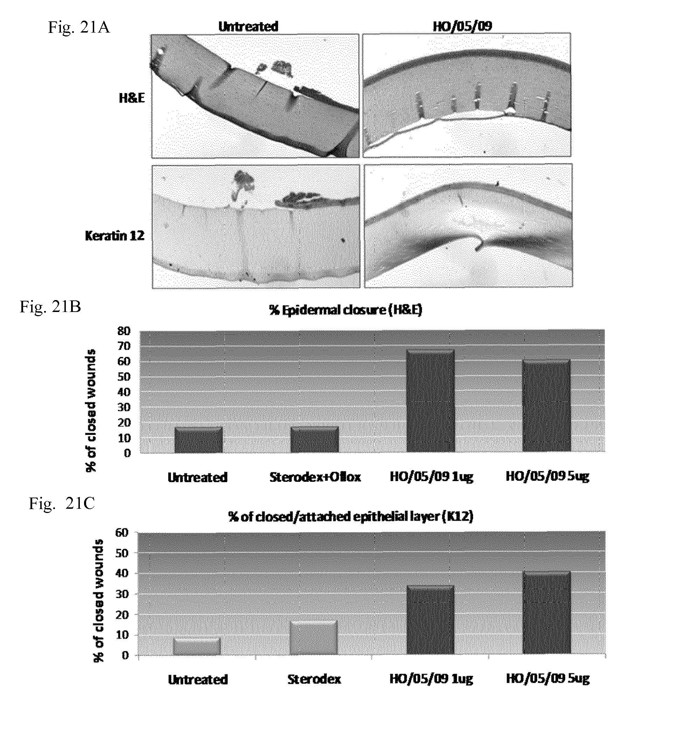

Figure 21 is a series of pictorial and graphical representations showing that HO/05/09 leads to maturation of corneal wound. Rabbits were subjected to chemical alkali burn wounding (6mm wide, NaOH IN for 20 seconds). Eyes were treated by three daily ocular instillations of treatments during 7 successive days. Figure 21A upper panel depicts histological H&E staining of the corneas. The epithelial layer in the untreated group is absent. Figure 21A lower panel depicts immunohistochemistry staining for K12. Figure 21B depicts the percentage of wounds that achieved full closure by H&E staining. Figure 21C depicts the percentage of wounds that achieved full closure by K12 staining.

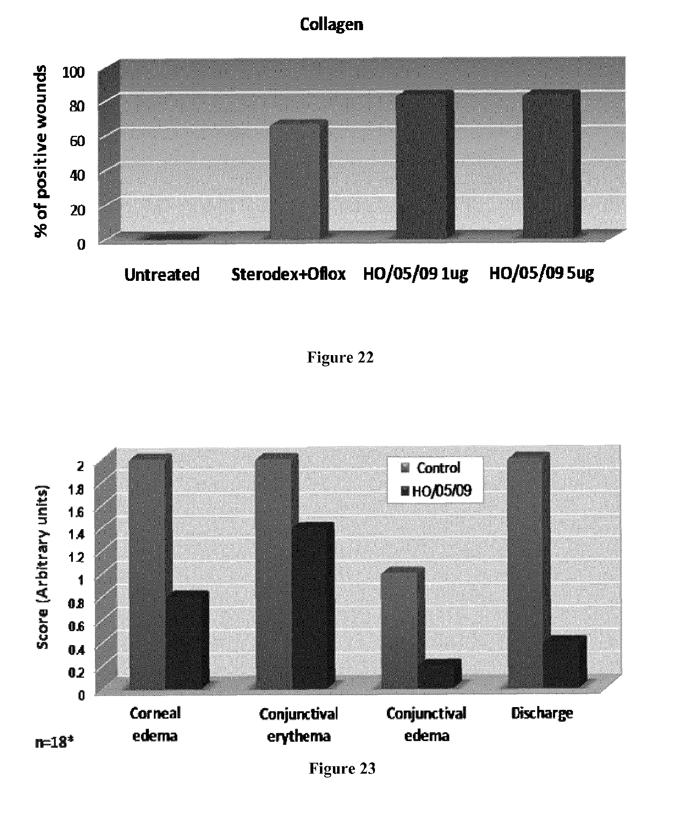

Figure 22 is a histogram showing that HO/05/09 enhances collagen deposition in corneal matrix. Rabbits were subjected to chemical alkali burn wounding (6mm wide, NaOH IN for 20 seconds). Eyes were treated by three daily ocular instillations of treatments during 7 successive days. The histogram summarizes histological collagen staining in the corneas.
Figure 23 is a histogram showing that HO/05/09 reduces selected parameters of corneal inflammatory response. Rabbits were subjected to chemical alkali burn wounding (6mm wide, NaOH IN for 20 seconds). Eyes were treated by three daily ocular instillations of treatments during 7 successive days. The histogram summarizes morphological assessment of inflammatory parameters by a clinical slit-lamp eye evaluation.

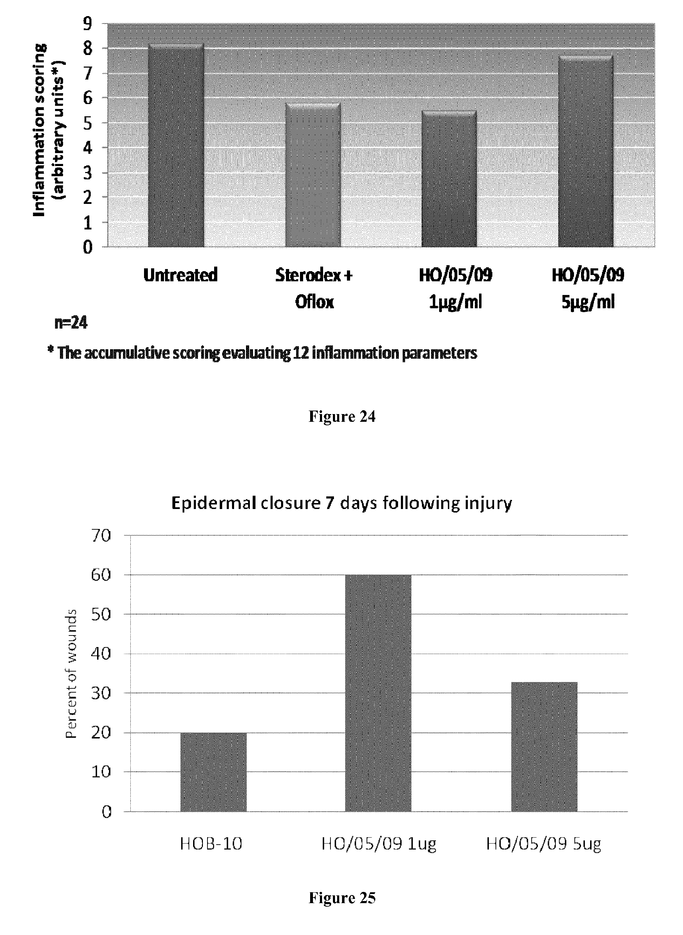

Figure 24 is a histogram showing that HO/05/09 reduces corneal inflammatory response by summarized parameters scoring. Rabbits were subjected to chemical alkali burn wounding (6mm wide, NaOH IN for 20 seconds). Eyes were treated by three daily ocular instillations of treatments during 7 successive days. The histogram summarizes accumulative scoring evaluating 12 inflammation parameters by a slit lamp examination.
Figure 25 is a histogram showing that HO/05/09 promotes re-epithelialization of corneal wound in mice. Mice were subjected to chemical alkali burn wounding (using Silver Nitrate applicators for 10 seconds). Eyes were treated by three daily ocular instillations of treatments during 7 successive days. Histological H&E staining of the corneas wounded area was performed and objective assessment of wound closure was presented graphically.

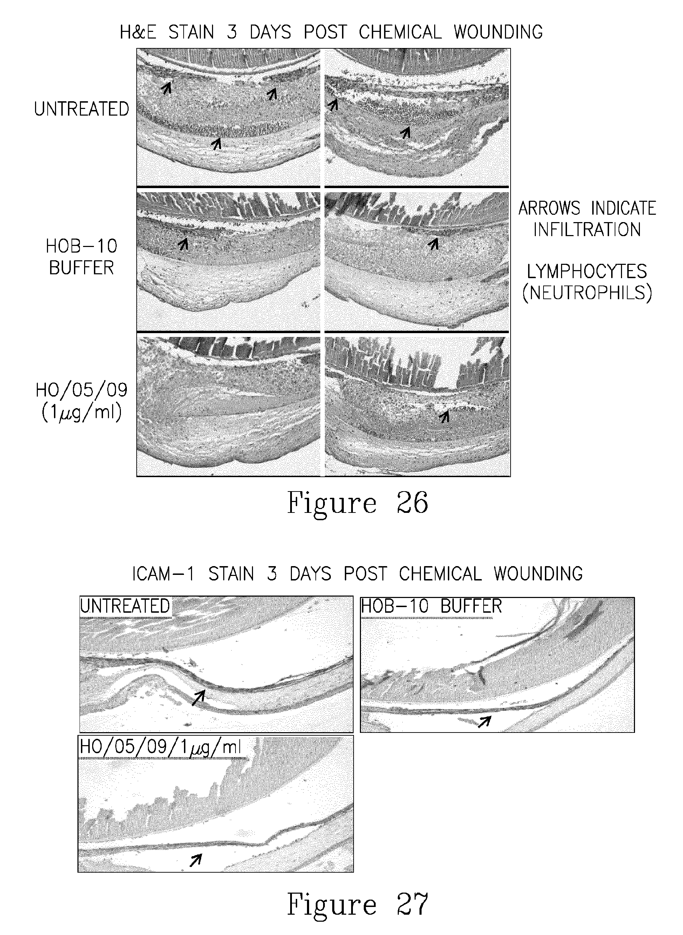

Figure 26 is a series of images showing that HO/05/09 reduces lymphocytes infiltration in corneal wound gap in mice. Mice were subjected to chemical alkali burn wounding (using Silver Nitrate applicators for 10 seconds). Eyes were treated by three daily ocular instillations of treatments during 7 successive days. Histological H&E staining of the corneas wounded area. Arrows indicated infiltrating lymphocytes.
Figure 27 is a series of images showing that HO/05/09 decreases I-CAM-1 levels in corneal wound in mice. Mice were subjected to chemical alkali burn wounding (using Silver Nitrate applicators for 10 seconds). Eyes were treated by three daily ocular instillations of treatments during 7 successive days. Immunohistochemistry staining for I-CAM-1.

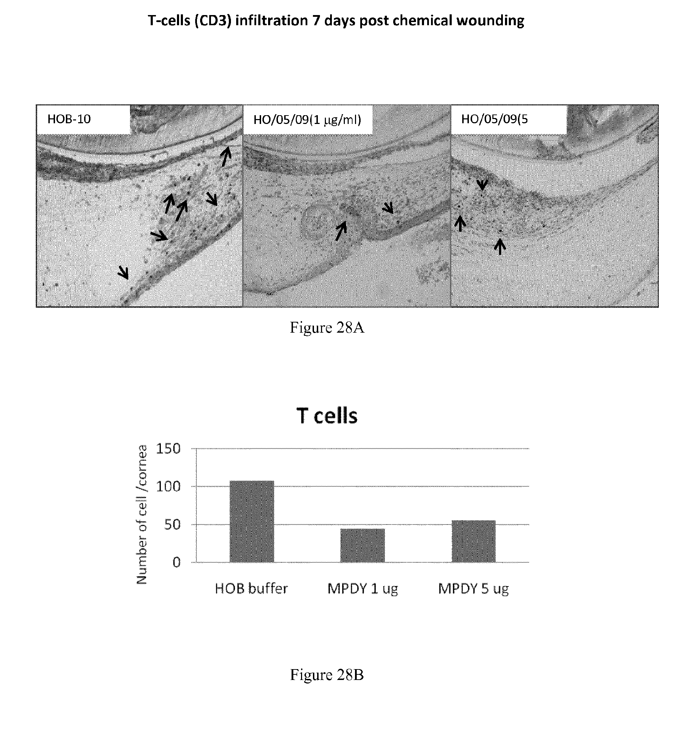

Figure 28 is a series of pictorial and graphical representations showing that HO/05/09 reduces T-cells recruitment to corneal wound in mice. Mice were subjected to chemical alkali burn wounding (using Silver Nitrate applicators for 10 seconds). Eyes were treated by three daily ocular instillations of treatments during 7 successive days. Figure 28A is images of immunohistochemistry staining for CD3 (T-cells). Figure 28B is a histogram summarizing graphical representation of T-cells per cornea.

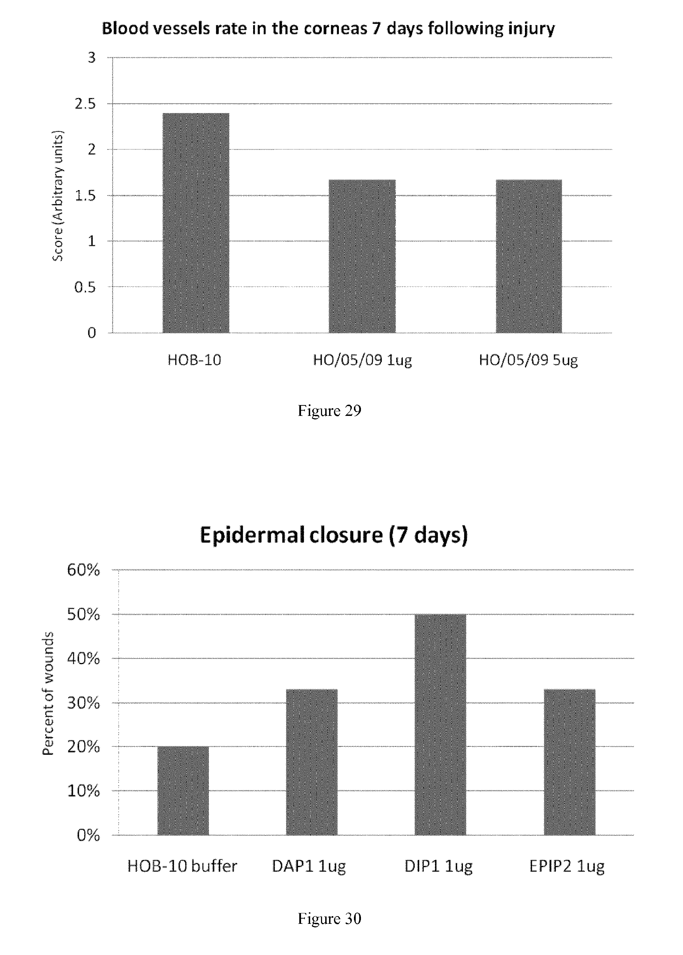

Figure 29 is a histogram showing that HO/05/09 decreases angiogenesis in corneal wound gap in mice. Mice were subjected to chemical alkali burn wounding (using Silver Nitrate applicators for 10 seconds). Eyes were treated by three daily ocular instillations of treatments during 7 successive days. Histological H&E staining of the corneas wounded area has been performed. Blood vessels in entire corneal area were assessed and the results are represented graphically in objective arbitrary units.
Figure 30 is a histogram showing that DAP-1 1µg/ml, DIP-1 1µg/ml and EPIP-2 1µg/ml promote re-epithelialization of corneal wound in mice. Mice were subjected to chemical alkali burn wounding (using Silver Nitrate applicators for 10 seconds). Eyes were treated by three daily ocular instillations of treatments during 7 successive days with DAP-1 1µg/ml, DIP-1 1µg/ml and EPIP-2 1µg/ml or with Formula 1 as control. Percentage of wounds achieved full epidermal closure at 7 days post wounding.

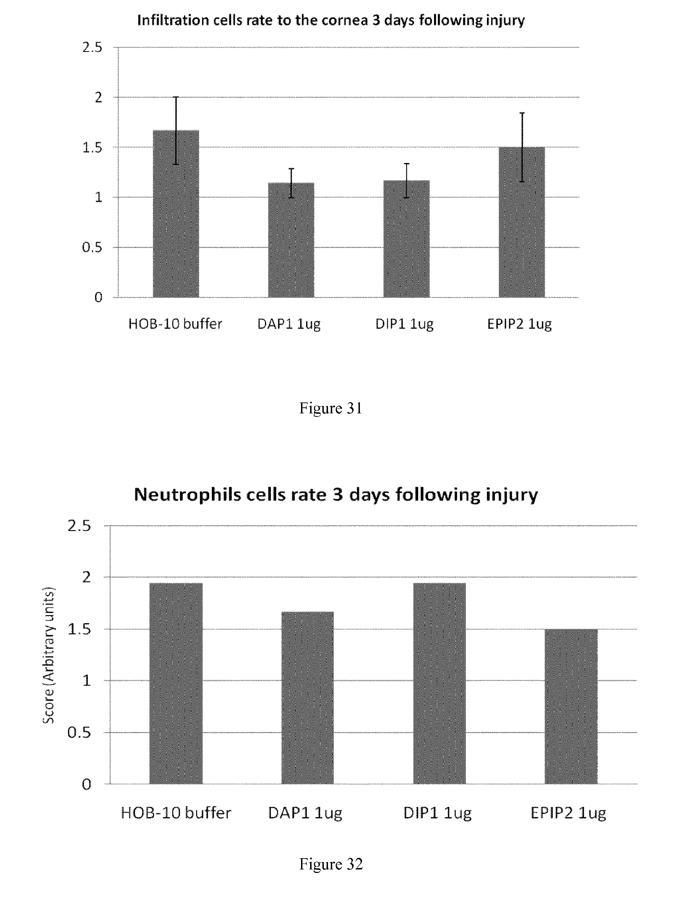

Figure 31 is a histogram showing that DAP-1 1µg/ml and DIP-1 1µg/ml reduce lymphocytes infiltration in corneal wound gap in mice. Mice were subjected to chemical alkali burn wounding (using Silver Nitrate applicators for 10 seconds). Eyes were treated by three daily ocular instillations of treatments during 3 successive days with DAP-1 1µg/ml, DIP-1 1µg/ml and EPIP-2 1µg/ml or with Formula 1 as control. Histological H&E staining of the corneas wounded area was performed and lymphocytes infiltration was arbitrarily assessed.
Figure 32 is a histogram showing that DAP-1 1µg/ml and EPIP-2 1µg/ml decrease neutrophils recruitment to the corneal wound gap in mice. Mice were subjected to chemical alkali burn wounding (using Silver Nitrate applicators for 10 seconds). Eyes were treated by three daily ocular instillations of treatments during 3 successive days with DAP-1 1µg/ml, DIP-1 1µg/ml and EPIP-2 1µg/ml or with Formula 1 as control. Histological sections of skin samples were stained with specific neutrophil staining kit (according to manufacturer). Neutrophils were counted per fixed field x200 and represented by arbitrary units.

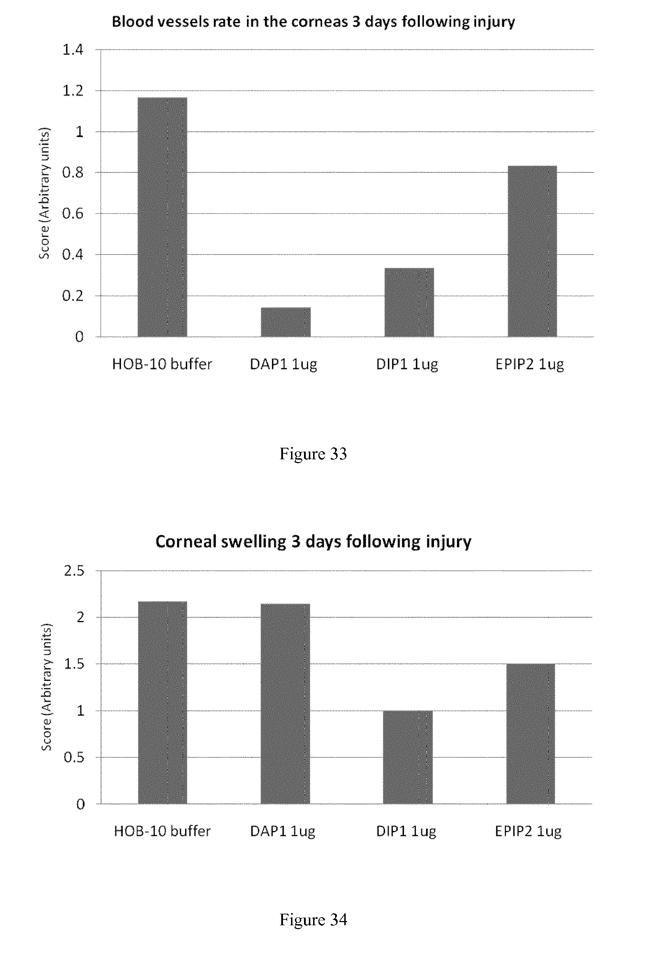

Figure 33 is a histogram showing that DAP-1 1µg/ml, DIP-1 1µg/ml and EPIP-2 1µg/ml reduce angiogenesis in corneal wound gap in mice. Mice were subjected to chemical alkali burn wounding (using Silver Nitrate applicators for 10 seconds). Eyes were treated by three daily ocular instillations of treatments during 3 successive days with DAP-1 1µg/ml, DIP-1 1µg/ml and EPIP-2 1µg/ml or with Formula 1 as control. Histological H&E staining of the corneas wounded area has been performed. Blood vessels in entire corneal area were assessed and the results are represented graphically in objective arbitrary units.
Figure 34 is a histogram showing that DIP-1 1µg/ml and EPIP-2 1µg/ml decrease pathological swelling of wounded cornea in mice. Mice were subjected to chemical alkali burn wounding (using Silver Nitrate applicators for 10 seconds). Eyes were treated by three daily ocular instillations of treatments during 3 successive days with DAP-1 1µg/ml, DIP-1 1µg/ml and EPIP-2 1µg/ml or with Formula 1 as control. Histological H&E staining of the corneas wounded area has been performed and corneal swelling was objectively assessed. The results are presented in arbitrary units.

### DETAILED DESCRIPTION

The present disclosure is based in part on the discovery of ophthalmic buffered compositions adapted for formulation of topically administrable suspensions useful in delivery of pharmaceutically active agents. The compositions enhance the pharmaceutical action of various pharmaceutically active agents as well as possessing therapeutic characteristics themselves.

The present disclosure also utilizes the knowledge that modulating distinct PKC isoforms is an effective tool to affect wound healing. As discussed in U.S. Patent Application Publication No. 2006/0258562, wound healing may be promoted by inhibiting or activating the expression and/or activity of a PKC isoform, such as PKC-α, PKC-ε, and PKC-δ. However, convenient delivery of agents to inhibit or activate such activity to ocular tissue requires specifically formulated ophthalmic solutions suitable for administration to the eye while promoting the pharmacological activity of the agent.

The present disclosure is based on the discovery of a specific ophthalmic formulation that itself exhibits therapeutic effects, and when combined with pharmaceutically active agents, enhances the mechanism of action of the agents to provide synergistic effects and increase the rate of healing of either administered alone.

Thus, in one aspect of the present disclosure, there is provided a method of accelerating or promoting healing of damaged ocular tissue or an ocular wound in a subject. The method includes administering a liquid ophthalmic composition as disclosed to an eye of a subject. The composition may be used alone or in combination with other pharmaceutical agents, such as a PKC isoform inhibitors or activator, or specifically a PKC-α, a PKC-ε inhibitor, a PKC-δ inhibitor or a PKC-δ activator.

In a related aspect, the ophthalmic composition may act as an artificial tear solution or optical lubricant promote wound healing or comfort. As such, the present disclosure provides a method of lubricating an eye of a subject. The method includes topically administering a liquid ophthalmic composition to an eye of the subject.

It is to be understood that this disclosure is not limited to particular compositions, methods, and experimental conditions described, as such compositions, methods, and conditions may vary. It is also to be understood that the terminology used herein is for purposes of describing particular embodiments only, and is not intended to be limiting, as the scope of the present inventions will be limited only in the appended claims.

The principles and operation of the methods and pharmaceutical compositions according to the present disclosure may be better understood with reference to the figures and accompanying descriptions.

As used in this specification and the appended claims, the singular forms "a", "an", and "the" include plural references unless the context clearly dictates otherwise. Thus, for example, references to "the method" includes one or more methods, and/or steps of the type described herein which will become apparent to those persons skilled in the art upon reading this disclosure and so forth.

Unless defined otherwise, all technical and scientific terms used herein have the same meaning as commonly understood by one of skill in the art to which this disclosure belongs. Although any methods and materials similar or equivalent to those described herein can be used in the practice or testing of the inventions, some preferred methods and materials are now described.

The present disclosure provides a liquid ophthalmic composition suitable for topical application to a mammalian eye. As used herein, the terms "ophthalmic solution", "liquid ophthalmic composition", "ophthalmic buffered composition" and permutations thereof are used interchangeably and refer to a liquid formulation that is ophthalmically acceptable or compatible with application to the eye and in certain embodiments forms a stock solution for addition of pharmaceutically active agents..

As used herein, the terms "ophthalmically acceptable composition" or "compatible with application to an eye" includes a composition which can be placed into a mammalian or human eye without causing any substantial discomfort, damage, or harm.

As used herein, the term "mammalian eye" refers to an eye of any animal in the order mammalia. Such animals include, but are not limited to horses, cats, dogs, rabbits, mice, goats, sheep, non-human primates and humans. Thus, the solutions are contemplated for use in veterinary applications as well as human use.

As used herein, the term "subject" refers to a mammalian subject. As such, treatment of any animal in the order mammalian is envisioned. Such animals include, but are not limited to horses, cats, dogs, rabbits, mice, goats, sheep, non-human primates and humans. Thus, the method of the present disclosure is contemplated for use in veterinary applications as well as human use.

The ophthalmic composition as described is formulated for ameliorating optical wounds.

As used herein, the term "wound" refers broadly to injuries to the epithelia initiated in any one of a variety of ways (for example, pressure, inflammation, wounds induced by trauma, cuts, ulcers, burns and the like) and with varying characteristics.

A "symptom" of a wound is any morbid phenomenon or departure from the normal in structure, function, or sensation, experienced by the subject and indicative of a wound.

The term "healing" in respect to a wound refers to a process to repair a wound as by restoring the wounded tissue or epithelia to a normal state or function.

The phrase "accelerating or promoting healing" refers to either the induction of the formation of granulation tissue of wound contraction and/or the induction of epithelialization (for example, the generation of new cells in the epithelium). Wound healing is conveniently measured by decreasing wound area.

The term "ocular" or "ocular tissue" is intended to refer to any tissue or cells pertaining to the eye. Additionally, the term "ocular" is used interchangeably with the term "eye".

The present disclosure contemplates treating all types of ocular wounds, including chronic wounds.

The term "chronic wound" refers to a wound that exhibits impaired healing parameters interfering with the physiological sequence of events. These wounds tend to prolong and/or halt healing time course, subjecting the wounds to further complications such as recurrent infections and necrosis.

"Treatment" of a subject herein refers to both therapeutic treatment and prophylactic or preventative measures. Those in need of treatment include those already with an optical wound as well as those in which it is to be prevented. Hence, the subject may be suffering from an optical wound or may be susceptible to an optical wound such as dry eye.

In various embodiments, the ophthalmic solution can be used for the treatment of a corneal ulceration wound, a retinopathy wound, a burn, an inflammation wound, a dry eye syndrome wound, a macular degeneration wound, a laceration, a surgical incision wound, or a post surgical adhesion wound.

The expression "effective amount" refers to an amount of a pharmaceutically active agent, such as an inhibitor or activator of a PKC isoform, such as the polypeptides of SEQ ID NOs: 1-18, that is effective for preventing, ameliorating or treating an optical wound. Such an effective amount will generally result in an improvement in the signs, symptoms and/or other indicators of the wound.

As used herein, the term "PKC isoform" as used herein encompasses all PKC isoforms including PKC-α, PKC-β, PKC-δ, PKC-ε, PKC-η, PKC-ζ, PKC-γ, PKC-θ, and PKC-λ.

The phrase "modulating expression and/or activity of a PKC isoform" relates to an increased or reduced expression and/or activity of a PKC isoform. Increase of the expression leads to increased production of the PKC isoform.

Overall, the results presented herein demonstrate that the ophthalmic compositions of the present invention are useful for facilitating ocular wound healing, as well as delivering pharmaceutically active agents to ocular tissue.

The ophthalmic composition may be used alone in treatment to facilitate wound healing. Thus, in one embodiment, the composition includes: a) 0.1 to 2.0% weight per volume (w/v) sodium chloride; b) 0.01 to 0.5% (w/v) potassium chloride; c) 0.01 to 1.0% (w/v) sodium acetate trihydrate; d) 0.01 to 1.0% (w/v) trisodium citrate dihydrate; and e) water, with the proviso that the composition does not include, or includes less than 0.03% (w/v) of both, or either of calcium chloride dihydrate or magnesium chloride hexahydrate.

Sodium chloride is an ionic compound with the formula NaCl. In an embodiment, an ophthalmic composition may include 0.1 to 2.0% (w/v) sodium chloride. In various embodiments, the composition may include from about 0.01 to 2.0%, 0.05 to 2.0%, 0.1 to 2.0%, 0.5 to 1.5%, 0.5 to 1.0%, 0.6 to 0.9%, 0.6 to 0.8%, 0.65 to 0.75%, or 0.7 to 0.75% (w/v) sodium chloride. In one embodiment, the composition includes about 0.70%, 0.71%, 0.73% or 0.74% (w/v) of sodium chloride.

The chemical compound potassium chloride is a metal halide salt composed of potassium and chlorine with the formula KCl. In an embodiment, an ophthalmic composition may include 0.01 to 0.5% (w/v) potassium chloride. In various embodiments, the composition may include from about 0.01 to 0.5%, 0.05 to 0.4%, 0.05 to 0.3%, 0.05 to 0.2%, .0.05 to 0.1%, 0.06 to 0.09%, 0.07 to 0.085%, or 0.08 to 0.085% (w/v) potassium chloride. In one embodiment, the composition includes about 0.08%, 0.081%, 0.082%, 0.083%, 0.084%, or 0.085% (w/v) of potassium chloride.

Sodium acetate trihydrate is the sodium salt of acetic acid having the formula C₂H₃NaO₂. In an embodiment, an ophthalmic composition may include 0.01 to 1.0% (w/v) sodium acetate trihydrate. In various embodiments, the composition may include from about 0.01 to 1.0%, 0.05 to 1.0%, 0.1 to 0.8%, 0.2 to 0.5% or 0.3 to 0.4% (w/v) sodium acetate trihydrate. In one embodiment, the composition includes about 0.35%, 0.36%, 0.37%, 0.38%, 0.39%, 0.40%, 0.41%, 0.42% or 0.43% (w/v) of sodium acetate trihydrate.

Sodium citrate dihydrate is an acid salt with the chemical formula NaH₂C₆H₅O₇. It will be understood that acid salts of monosodium, disodium and trisodium formulas may be used in accordance with the present invention. It will also be understood that salts of differing hydration may be used in accordance with the present invention. In an embodiment, an ophthalmic composition may include 0.01 to 1.0 (w/v) sodium citrate dihydrate or trisodium citrate dihydrate. In various embodiments, the composition may include from about 0.01 to 1.0%, 0.05 to 1.0%, 0.1 to 0.8%, 0.1 to 0.5%, 0.1 to 0.4% 0.1 to 0.3%, 0.1 to 0.2% or 0.15 to 0.2% (w/v) sodium citrate dihydrate or trisodium citrate dihydrate. In one embodiment, the composition includes about 0.15%, 0.16%, 0.17%, 0.18%, 0.19% or 0.2% (w/v) of sodium citrate dihydrate or trisodium citrate dihydrate.

It will be understood that equivalent amounts of non-hydrate salts may be used in accordance with the invention. It will also be understood that salts of differing hydration may be used in accordance with the present invention. For example, magnesium chloride hydrates of the general formula MgCl₂(H₂O)ₓ wherein x= from 1-5 or 7-12 may be used in place of the hexahydrate salt, and similar substitutions may be made with the other preferred hydrate salts of the buffer composition.

The ophthalmic composition may be used alone in treatment to facilitate wound healing or may be combined with one or more pharmaceutically active agents. Thus, in one embodiment, the composition includes: a) 0.1 to 2.0% (w/v) sodium chloride; b) 0.01 to 0.5% (w/v) potassium chloride; c) 0.01 to 1.0% (w/v) sodium acetate trihydrate; d) 0.01 to 1.0% (w/v) trisodium citrate dihydrate; e) water; and a pharmaceutically active agent.

In various embodiments the pharmaceutically active agent is a PKC-isoform inhibitor or activator, such as a PKC-α inhibitor, a PKC-ε inhibitor, a PKC-δ inhibitor or a PKC-δ activator. The term "activator" is used herein to describe a molecule that enhances expression and/or activity of a PKC isoform. The term "inhibitor" is used herein to describe a molecule that inhibits expression and/or activity of a PKC isoform. Among others, the phosphoryl transfer region, the pseudosubstrate domain, the phorbolester binding sequences, and the phosphorylation sites may be targets for modulation of isoenzyme-specific PKC activity.

The "pseudosubstrate region" or autoinhibitory domain of a PKC isoform is herein defined as a consensus sequence of substrates for the kinase with essentially no phosphorylatable residue. The pseudosubstrate domain is based in the regulatory region, closely resembling the substrate recognition motif, which blocks the recognition site and prevents phosphorylation. Thus, inhibitory polypeptides of PKC isoforms, such as the polypeptides of the present disclosure, are obtained as by replacing a phosphorylatable residue of serine (S) or tyrosine (T) by alanine (A).

In various embodiments, the PKC isoform inhibitor or activator is a polypeptide. Without being limited to a particular theory, it has been discovered that the mechanism of action of polypeptide PKC inhibitors and activators is enhanced when combined and administered with the ophthalmic solution as described. The ophthalmic buffered solution provides treatment as well as accommodating the PKC inhibitor or activator and works synergistically by enhancing the mechanism of action of the inhibitor to increase the rate of healing that is provided by either the ophthalmic solution or active agent alone or in combination with other buffer solutions.

In various embodiments, the inhibitors of PKC isoforms are inhibitors of the pseudosubstrate region of PKC and are polypeptides, while the activators of PKC isoforms are also polypeptides. The terms "polypeptide", "peptide", or "protein" are used interchangeably herein to designate a linear series of amino acid residues connected one to the other by peptide bonds between the alpha-amino and carboxy groups of adjacent residues.

In various embodiments, Examples of polypeptide PKC activators and inhibitors that can be used include, without being limited to, peptides of SEQ ID NOs: 1-6, 12, 14 and 17, as shown in Table 1 or physiologically acceptable salts thereof, as well as the peptides of SEQ ID NOs: 7-11, 13, 15, 16 and 18 of Table 1 which are shown having particular modifications or terminal protecting groups.

**Table 1: PKC Isoform Inhibitor and Activator Peptides**

| **Amino Acid Sequence** | **SEQ ID NO** |
|---|---|
| **PKC-α Inhibitors** | |
| Phe-Ala-Arg-Lys-Gly-Ala | **1** |
| Phe-Ala-Arg-Lys-Gly-Ala-Leu-Arg-Gln | **2** |
| Phe-Ala-Arg-Lys-Gly-Ala-Leu-Arg-Gln | **3** |
| Phe-Ala-Arg-Lys-Gly-Ala-Leu | **4** |
| Phe-Ala-Arg-Lys-Gly-Ala-Arg-Gln | **5** |
| Thr-Leu-Asn-Pro-Gln-Trp-Glu-Ser | **6** |
| Myristoyl-Phe-Ala-Arg-Lys-Gly-(13C315N)Ala-Leu-Arg-Gln-OH | **7** |
| H-Phe-Ala-Arg-Lys-Gly-Ala-Leu-Arg-Gln-OH | **8** |
| Myristoyl-Phe-Ala-Arg-Lys-Gly-Ala-Leu-OH-trifluoracetate salt | **9** |
| H-Thr-Leu-Asn-Pro-Gln-Trp-Glu-Ser-OH | **10** |
| Palmitoyl-Phe-Ala-Arg-Lys-Gly-Ala-Arg-Gln-OH | **11** |

| **PKC-ε Inhibitors** | |
|---|---|
| Pro-Tyr-I le-Ala-Leu-Asn-V al-Asp | **12** |
| H-Pro-Tyr-Ile-Ala-Leu-Asn-Val-Asp-OH | **13** |

| **PKC**-**δ Inhibitors** | |
|---|---|
| Ser-Phe-Asn-Ser-Tyr-Glu-Leu-Gly-Ser-Leu | **14** |
| Ser-Phe-Asn-Ser-Tyr-Glu-Leu-Gly-Ser-Leu-OH | **15** |
| Myristoyl-Ser-Phe-Asn-Ser-Tyr-Glu-Leu-Gly-Ser-Leu-OH | **16** |

| **PKC**-**δ Activators** | |
|---|---|
| Met-Arg-Ala-Ala-Glu-Ala-Ala-Ala-Ala-Glu-Pro-Met | **17** |
| H-Met-Arg-Ala-Ala-Glu-Ala-Ala-Ala-Ala-Glu-Pro-Met-OH | **18** |

In various embodiments, the polypeptide PKC inhibitors or activators typically contain between 6 and 12 amino acids, but may be longer or shorter in length. In various embodiments, a polypeptide PKC inhibitor or activator may range in length from 6 to 45, 6 to 40, 6 to 35, 6 to 30, 6 to 25, 6 to 20, 6 to 15, or 6 to 10 amino acids. In one embodiment the polypeptide includes 6, 7, 8, 9, 10, 11, 12, 13, 14 or 15 amino acids.

In general, polypeptide PKC-α inhibitors include the common motif sequence Phe-Ala-Arg-Lys-Gly-Ala (SEQ ID NO: 1). Alternatively, in another embodiment, PKC-α inhibitors include the common motif sequence Thr-Leu-Asn-Pro-Gln-Trp-Glu-Ser (SEQ ID NO: 6).

While the polypeptide PKC inhibitors and activators may be defined by exact sequence or motif sequences, one skilled in the art would understand that polypeptides that have similar sequences may have similar functions. Therefore, polypeptides having substantially the same sequence or having a sequence that is substantially identical or similar to a PKC inhibitor or activator of Table 1 are intended to be encompassed. As used herein, the term "substantially the same sequence" includes a polypeptide including a sequence that has at least 60+% (meaning sixty percent or more), preferably 70+%, more preferably 80+%, and most preferably 90+%, 95+%, or 98+% sequence identity with the sequences defined by SEQ ID NOs: 1-18 and retain 60+%, preferably 70+%, more preferably 80+%, and most preferably 90+%, 95+%, or 98+% of the PKC isoform inhibitory or activation activity as compared to the polypeptides of SEQ ID NOs: 1-18.

A further indication that two polypeptides are substantially identical is that one polypeptide is immunologically cross reactive with that of the second. Thus, a polypeptide is typically substantially identical to a second polypeptide, for example, where the two peptides differ only by conservative substitutions.

The term "conservative substitution" is used in reference to proteins or peptides to reflect amino acid substitutions that do not substantially alter the activity (for example, antimicrobial activity) of the molecule. Typically conservative amino acid substitutions involve substitution of one amino acid for another amino acid with similar chemical properties (for example, charge or hydrophobicity). The following six groups each contain amino acids that are typical conservative substitutions for one another: 1) Alanine (A), Serine (S), Threonine (T); 2) Aspartic acid (D), Glutamic acid (E); 3) Asparagine (N), Glutamine (Q); 4) Arginine (R), Lysine (K) 5) Isoleucine (I), Leucine (L), Methionine (M), Valine (V); and 6) Phenylalanine (F), Tyrosine (Y), and Tryptophan (W).

The term "amino acid" is used in its broadest sense to include naturally occurring amino acids as well as non-naturally occurring amino acids including amino acid analogs. In view of this broad definition, one skilled in the art would know that reference herein to an amino acid includes, for example, naturally occurring proteogenic (L)-amino acids, (D)-amino acids, chemically modified amino acids such as amino acid analogs, naturally occurring non-proteogenic amino acids such as norleucine, and chemically synthesized compounds having properties known in the art to be characteristic of an amino acid. As used herein, the term "proteogenic" indicates that the amino acid can be incorporated into a protein in a cell through a metabolic pathway.

The terms "identical" or percent "identity" in the context of two polypeptide sequences, refer to two or more sequences or subsequences that are the same or have a specified percentage of amino acid residues that are the same, when compared and aligned for maximum correspondence, as measured using a sequence comparison algorithm or by visual inspection.

The phrase "substantially identical," in the context of two polypeptides, refers to two or more sequences or subsequences that have at least 60+%, preferably 80+%, most preferably 90-95+% amino acid residue identity, when compared and aligned for maximum correspondence, as measured using a sequence comparison algorithm or by visual inspection.

As is generally known in the art, optimal alignment of sequences for comparison can be conducted, for example, by the local homology algorithm of Smith & Waterman ((1981) Adv Appl Math 2:482), by the homology alignment algorithm of Needleman & Wunsch ((1970) J Mol Biol 48:443), by the search for similarity method of Pearson & Lipman ((1988) Proc Natl Acad Sci USA 85:2444), by computerized implementations of these algorithms by visual inspection, or other effective methods.

Polypeptide PKC inhibitors or activators may have modified amino acid sequences or non-naturally occurring termini modifications. Modifications to the peptide sequence can include, for example, additions, deletions or substitutions of amino acids, provided the polypeptide produced by such modifications retains PKC isoform inhibitory or activation activity. Additionally, the polypeptides can be present in the formulation with free termini or with amino-protected (such as N-protected) and/or carboxy-protected (such as C-protected) termini. Protecting groups include: (a) aromatic urethane-type protecting groups which include benzyloxycarbonyl, 2-chlorobenzyloxycarbonyl, 9-fluorenylmethyloxycarbonyl, isonicotinyloxycarbonyl and 4-methoxybenzyloxycarbonyl; (b) aliphatic urethane-type protecting groups which include t-butoxycarbonyl, t-amyloxycarbonyl, isopropyloxycarbonyl, 2-(4-biphenyl)-2-propyloxycarbonyl, allyloxycarbonyl and methylsulfonylethoxycarbonyl; (c) cycloalkyl urethane-type protecting groups which include adamantyloxycarbonyl, cyclopentyloxycarbonyl, cyclohexyloxycarbonyl and isobornyloxycarbonyl; (d) acyl protecting groups or sulfonyl protecting groups. Additional protecting groups include benzyloxycarbonyl, t-butoxycarbonyl, acetyl, 2-propylpentanoyl, 4-methylpentanoyl, t-butylacetyl, 3-cyclohexylpropionyl, n-butanesulfonyl, benzylsulfonyl, 4-methylbenzenesulfonyl, 2-naphthalenesulfonyl, 3-naphthalenesulfonyl and 1-camphorsulfonyl.

In various embodiments, the polypeptide PKC inhibitors or activators are N-acylated, preferably by an acyl group derived from a C12-C20 fatty acid, such as C14 acyl (myristoyl) or C16 acyl (palmitoyl).

In one embodiment, the PKC-α inhibitor is N-acylated, preferably by an acyl group derived from a C12-C20 fatty acid, such as C14 acyl (myristoyl) or C16 acyl (palmitoyl). In an exemplary embodiment, the polypeptide is an N-myristoylated peptide defined by SEQ ID NO: 2 (herein referred to as MPDY-1).

In one embodiment, the PKC-δ inhibitor is N-acylated, preferably by an acyl group derived from a C12-C20 fatty acid, such as C14 acyl (myristoyl) or C16 acyl (palmitoyl). In an exemplary embodiment, the polypeptide is an N-myristoylated peptide defined by SEQ ID NO: 16 (herein referred to as DIP-1).

In one embodiment the PKC-δ activator is the polypeptide of SEQ ID NO: 18 (herein referred to a DAP-1).

In one embodiment the PKC-δ inhibitor is the polypeptide of SEQ ID NO: 15 (herein referred to a DIP-2).

In one embodiment the PKC-ε inhibitor is the polypeptide of SEQ ID NO: 13 (herein referred to an EPIP-2).

In one embodiment, the composition includes a polypeptide PKC isoform inhibitor, including a PKC-α inhibitor, a PKC-ε inhibitor, a PKC-δ inhibitor or a PKC-δ activator.

In various embodiments, the pH of the ophthalmic solution is neutral to slightly alkaline. It is preferred that the ophthalmic solution have a pH between about 5 to 9, 6.5 and 8.0, 6.8 and 7.8, 7.1 and 7.8 or 7.1 and 7.5. In one embodiment, the pH is about 7.0, 7.1, 7.2, 7.3, 7.4, 7.5, 7.6, 7.7 or 7.8.

Methods of preparing buffer solutions are well known to those of skill in the art and can be found, for example in any of a number of standard laboratory manuals (see, Sambrook et al., Molecular Cloning: A Laboratory Manual, Cold Spring Harbor Press (1989)). In an exemplary embodiment, the ophthalmic buffered solution is formulated as a sterile solution.

The concentrations of salts included in the ophthalmic solution broadly establish the tonicity of the solution. The tonicity of the solution is usually about 220-320 milliosmoles per kilogram solution (mOsm/kg) to render the solution compatible with ocular tissue. In one embodiment, the solution has an osmolality of about 300 mOsm/kg.

Typically, particularly when used as an artificial tear, the ophthalmic solution has a viscosity from about 1 to about 50, 100, 500, 1000, 2000, 3000, 4000, or 5000 centipoise (cps). As a solution, the subject composition is usually dispensed in the eye in the form of an eye drop. It should be understood, however, that the subject composition may also be formulated as a viscous liquid (such as, viscosities from 50 to 50,000 cps), gel, or ointment.

The composition may include other additives. For example, the composition may include one or more of the following: a buffering agent, preservative, tonicity adjusting agent, demulcent, wetting agent, surfactant, solubilizing agent, stabilizing agent, comfort enhancing agent, emollient, pH adjusting agent, lubricant, aggregation inhibitory agent, charge modifying agent, degradative enzyme inhibitor, membrane penetration enhancer, sequestering agent (chelating agent), vasodilator or viscosity adjusting agent.

While the buffer solution itself may be considered a tonicity adjusting agent and a pH adjusting agent that broadly maintains the ophthalmic solution at a particular ion concentration and pH, additional tonicity adjusting agents can be added to adjust the final tonicity of the solution. Such tonicity adjusting agents are well known to those of skill in the art and include, but are not limited to mannitol, sorbitol, dextrose, sucrose, urea, and glycerin. Also, various salts, including halide salts of a monovalent cation are known to adjust tonicity. In embodiments where a tonicity adjusting agent is used, the ophthalmic solution may contain a single agent or a combination of different tonicity adjusting agents.

In various embodiments, the ophthalmic solution may further include one or more surfactants. Suitable surfactants include cationic, anionic, non-ionic or amphoteric surfactants. Preferred surfactants are neutral or nonionic surfactants.

Various nonionic surfactants are well known and suitable for use. Nonionic surfactants include non-ionic block copolymers, such as poly(oxyethylene)-poly(oxypropylene) block copolymers (also known as poloxamers). Such copolymers are known commercially and are produced in a wide range of structures and molecular weights with varying contents of ethylene oxide. These non-ionic surfactants are non-toxic, stable and readily dispersible in aqueous systems and are compatible with a wide variety of formulations and other ingredients for ophthalmic preparations. Further, poloxamers are well suited to ophthalmic applications as they generally afford minimal or no eye irritation. One class of poloxamer well suited for use in the ophthalmic solutions is a specific class of polyethyleneoxy-polypropyleneoxy block copolymer adducts of ethylene diamine (also known as poloxamine), which agents are both effective at cleaning and exhibit minimal or no eye irritation.

Examples of suitable surfactants include, but are not limited to, polyethylene glycol esters of fatty acids, polyoxypropylene ethers of C12-C18 alkanes and polyoxyethylene, polyoxypropylene block copolymers of ethylene diamine (such as, poloxamine). Other examples include poloxamer 182LF, poloxamer 188, poloxamer 331, poloxamer 407NF, sodium lauryl sulfate, pluronic F-127, Povidone (Sigma), PVP k-30, hydroxyethyl cellulose, NF and Tyloxapol (Sigma).

In certain embodiments, the shelf-life of the ophthalmic solution may be enhanced by the inclusion of one or more cation chelating agents, more preferably a chelator of divalent cations. Chelating agents are well known to those of skill in the art. Examples of chelating agents include, but are not limited to, ethylenediaminetetraacetic acid (EDTA) and its salts (for example, disodium), ethylenebis(oxyethylenenitrilo)tetraacetic acid (EGTA), and 2,2'-(ethylenediimino)-dibutyric acid EDBA which are normally employed in amounts from about 0.01 to about 0.2% (w/v). Other known chelating agents (or sequestering agents) such as certain polyvinyl alcohols may also be employed.

In certain embodiments, the ophthalmic solution can optionally include one or more species of divalent cation. Divalent cations include, but are not limited to Mg²⁺, Ca²⁺, Zn²⁺, Fe²⁺ and Ba²⁺.

In certain embodiments, the ophthalmic solution includes a chelating agent (such as, disodium EDTA) and/or additional microbicide, polyhexamethylene biguanide (PHMBO), N-alkyl-2-pyrrolidone, chlorhexidine, polyquatemium-1, hexetidine, bronopol, alexidine, low concentrations of hydrogen peroxide, and ophthalmologically acceptable salts thereof.

Additional microbicides and antimicrobial agents may include, but are not limited to chlorhexidine, thimerosal, boric acid, borate salts, potassium sorbate and sodium sorbate, quaternary ammonium salts, formaldehyde donors, benzethonium chloride, benzoic acid, benzyl alcohol, butylparaben, cetylpyridinium chloride, chlorobutanol, chlorocresol, cresol, dehydroacetic acid, ethylparaben, methylparaben, methylparaben sodium, phenol, phenylethyl alcohol, phenylmercuric acetate, phenylmercuric nitrate, polyquad, potassium benzoate, propylparaben, propylparaben sodium, sodium benzoate, sodium dehydroacetate, sodium propionate, sorbic acid, sodium perborate, thymol, and antimicrobial polypeptides (such as, a crecropin, a defensin, and a magainin) and mixtures thereof. Antimicrobial agents are typically used in a concentration ranging from about 0.01 to 2.5% (w/v).

In various embodiments, the ophthalmic solution may optionally include a demulcent. Demulcents are substances that soothe irritated tissue, particularly mucous membranes. Demulcents (or humectants) are used for lubricating mucous membrane surfaces and for relieving dryness and irritation. The term "demulcent" refers to a water-soluble polymer, which is applied topically to the eye to protect and lubricate mucous membrane surfaces and relieve dryness and irritation. Within this meaning, the term "wetting agent" is also commonly used. Furthermore, it will be understood that some constituents possess several functional attributes. For example, cellulose derivatives are common demulcents, but are also used as "viscosity increasing agents". Similarly, glycerin is a known demulcent but is also used as a "tonicity adjusting agent". Examples of widely used demulcents include: polyvinyl alcohol, polyvinyl pyrrolidone, cellulose derivatives and polyethylene glycol.

Examples of demulcents approved by the U.S. Food & Drug Administration include cellulose derivatives, such as carboxymethylcellulose sodium, hydroxyethyl cellulose, hydroxypropyl methylcellulose, and methylcellulose; dextran 70; gelatin; polyols, such as glycerin, polyethylene glycol 300, polyethylene glycol 400, polysorbate 80, propylene glycol, polyvinyl alcohol, and povidone (polyvinyl pyrrolidone).

Specific examples of known ophthalmic compositions comprising various demulcents are known to those of skill in the art. For example, U.S. Pat. No: 5,591,426 discloses an ophthalmic solution useful as an artificial tear. The reference includes a specific example of a borate buffered, preserved (such as, benzalkonium chloride), aqueous solution including the following three demulcents: 1) glycerin, 2) polyvinyl pyrrolidone, and 3) a cellulose derivative, such as, hydroxypropyl methyl cellulose. U.S. Pat. No. 5,106,615 discloses isotonic humectant eye drops including glycerin, polyethylene glycol, or propylene glycol with an anionic polymer such as Carbomer 941. Other references disclose the use of various combinations of demulcents including, but not limited to propylene glycol, polysorbate 80, polyvinyl pyrrolidone, polyethylene oxide, polystyrene sulfonate, and polyacrylamide, hydroxy ethyl cellulose, polyethylene glycol 6000, and dextrose (see, U.S. Patent Numbers: 4,029,817, 3,767,788; 3,767,789; 3,856,919; 3,907,985; 3,920,810; 3,947,573; 3,987,163, 3,549,747, 4,131,651, 4,120,949, and 4,409,205).

In certain embodiments, the ophthalmic solutions can optionally include viscosity adjusting agents (for example, particularly when the ophthalmic solution is intended to act as a lubricant (such as an artificial tear)). Suitable viscosity adjusting agents for administration to an eye are well known to those of skill in the art. In particular, cellulose derivatives are commonly used to increase viscosity, and as such, offer other advantages. Specific cellulose derivatives include, but are not limited to hydroxypropyl methyl cellulose, carboxymethyl cellulose, methyl cellulose, hydroxyethyl cellulose, and the like.

While one of skill in the art would appreciate that the present ophthalmic compositions may be utilized for local delivery of a pharmaceutically active agent to ameliorate ocular wounds, agents may also be delivered systemically via application of the solution to the eye. To assist in adsorption of a pharmaceutically active agent via mucosal membranes of ocular tissue, the ophthalmic composition may include a membrane penetration-enhancing agent. Agents that may provide membrane penetration enhancement include, for example, a surfactant, a bile salt, a phospholipid additive, a mixed micelle, liposome, or carrier, an alcohol, an enamine, an nitric oxide donor compound, a long-chain amphipathic molecule, a small hydrophobic penetration enhancer, a sodium or a salicylic acid derivative, a glycerol ester of acetoacetic acid, a cyclodextrin or beta-cyclodextrin derivative, a medium-chain fatty acid, a chelating agent, an amino acid or salt thereof, an N-acetylamino acid or salt thereof, and enzymes degradative to a selected membrane component.

In one aspect, the ophthalmic composition may be used as an ocular lubricant or artificial tear. As such, the present disclosure provides a method of lubricating an eye. The method includes topically administering a liquid ophthalmic composition to an eye of a subject.

In another aspect, the ophthalmic composition includes one or more pharmaceutically active agents. As discussed herein, one of skill in the art would appreciate that the pharmaceutically active agent may be administered for local effects (such as, wound treatment) or systemic effects via adsorption into the circulatory system of a subject.

In general, the ophthalmic composition may be used to administer various pharmaceutically active compounds to the eye. Such pharmaceuticals may include, but are not limited to, anesthetic, astringent, anti-hypertensive, anti-glaucoma, neuro-protective, anti-allergy, muco-secretagogue, angiostatic, anti-microbial, pain-relieving or anti-inflammatory agents.

In certain embodiments, the pharmaceutically active agent is an ophthalmic drug. Such drugs include, but are not limited to: anti-glaucoma agents, such as beta-blockers including timolol, betaxolol, levobetaxolol, carteolol, miotics including pilocarpine, carbonic anhydrase inhibitors, prostaglandins, seretonergics, muscarinics, dopaminergic agonists, adrenergic agonists including apraclonidine and brimonidine; anti-angiogenesis agents; anti-infective agents including quinolones such as ciprofloxacin, and aminoglycosides such as tobramycin and gentamicin; non-steroidal and steroidal anti-inflammatory agents, such as suprofen, diclofenac, ketorolac, rimexolone and tetrahydrocortisol; growth factors, such as EGF; immunosuppressant agents; and anti-allergic agents including olopatadine. The ophthalmic drug may be present in the form of a pharmaceutically acceptable salt, such as timolol maleate, brimonidine tartrate or sodium diclofenac. Compositions may also include combinations of ophthalmic drugs, such as combinations of (i) a beta-blocker, such as betaxolol and timolol, and (ii) a prostaglandin such as latanoprost; 15-keto latanoprost; travoprost; and unoprostone isopropyl.

In various embodiments, a pharmaceutically active agent may be any type of molecule. For example, the pharmaceutically active agent may be an oligonucleotide, hormone, steroid, corticosteroid, lipid, polypeptide, peptidomimetic, peptoid such as a vinylogous peptoid, or chemical compound such as organic molecules or small organic molecules. One of skill in the art would appreciate that any given pharmaceutically active agent may be derived synthetically or naturally.

Further, in addition to PKC inhibitory polypeptides, any other types of polypeptide may be formulated, such as any other medically or diagnostically useful polypeptide. The ophthalmic compositions may be combined with any polypeptide, although the degree to which the polypeptide benefits are improved may vary according to the molecular weight and the physical and chemical properties of the polypeptide. For example, the polypeptide may be a growth factor such as PDGF, EGF, TGF-β, KGF, ECGF, IGF1, or PDGF-BB. The polypeptide may also be insulin. The insulin may be recombinant or from a natural source such as human insulin or a non-human mammal insulin that is suitable for human use such as porcine insulin. One skilled in the art would understand that insulin analogs, such as chemically or amino acid modified analogs may also be used. Examples of insulin analogs include, but are not limited to, NPH insulin, insulin lispro, insulin aspart, insulin glulisine, insulin glargine and insulin detemir.

In another embodiment, the pharmaceutically active agent is an oligonucleotide. The terms "polynucleotide" and "oligonucleotide" are used interchangeably herein to refer to nucleic acid molecules. A number of pharmaceutically active oligonucleotides are known in the art which may be utilized in the presently described ophthalmic composition. Such oligonucleotides may include, antisense molecules, siRNAs, or the like.

While the ophthalmic solutions may preferably be formulated as ready for use aqueous solutions, alternative formulations are contemplated within the scope of this disclosure. Thus, for example, the ophthalmic solution can be lyophilized or otherwise provided as a dried powder or tablet ready for dissolution in water (such as, deionized or distilled).

In one embodiment, formulation of the compositions in dry powder or tablet formats involves protection of a peptide pharmaceutical agent present in the composition. Effective formulations typically involve processing and formulating the protein, and other agents if present, so that the protein's conformation and biological activity are maintained throughout processing and during prolonged release from the dry form. Sustained protein packaging systems can be achieved with a variety of known microsphere delivery systems often used for *in vivo* delivery of protein therapeutics.

One microsphere fabrication process is described in U.S. Patent No. 5,019,400 and was specifically designed to achieve a high protein encapsulation efficiency while maintaining protein integrity. The process includes: (i) preparation of freeze-dried protein particles from bulk protein by spray freeze-drying the drug solution with stabilizing excipients; (ii) preparation of a drug-polymer suspension followed by sonication or homogenization to reduce the drug particle size; (iii) production of frozen drug-polymer microspheres by atomization into liquid nitrogen; (iv) extraction of the polymer solvent with ethanol; and (v) filtration and vacuum drying to produce the final dry-powder product. The resulting powder contains the solid form of the protein, which is homogeneously and rigidly dispersed within porous polymer particles. The polymer most commonly used in the process, poly(lactide-co-glycolide) (PLG), is both biocompatible and biodegradable.

In another embodiment, one or more components of the solution can be provided as a "concentrate", for example, in a storage container (such as, in a premeasured volume) ready for dilution, or in a soluble capsule ready for addition to a volume of water.

In still another embodiment, this disclosure provides kits that utilize one or more of the ophthalmic solutions described herein. Thus, for example, kits may include one or more containers containing one or more ophthalmic solutions, tablets, or capsules as described. The kits may be designed to include instructional materials containing directions (for example, protocols) disclosing use of the ophthalmic solutions provided therein. While the instructional materials typically comprise written or printed materials they are not limited to such. Any medium capable of storing such instructions and communicating them to an end user is contemplated. Such media include, but are not limited to electronic storage media (for example, magnetic discs, tapes, cartridges, chips), optical media (for example, CD ROM), and the like. Such media may include addresses to internet sites that provide such instructional materials.

The term "instructions" or "package insert" is used to refer to instructions customarily included in commercial packages of therapeutic products, that contain information about the indications, usage, dosage, administration, contraindications, other therapeutic products to be combined with the packaged product, and/or warnings concerning the use of such therapeutic products, and the like.

It will be understood, that the specific dose level and frequency of dosage for any particular subject in need of treatment may be varied and will depend upon a variety of factors including the activity of the pharmaceutically active agent employed, the metabolic stability and length of action of that compound, the age, body weight, general health, sex, diet, mode and time of administration, rate of excretion, drug combination, the severity of the particular condition, and the host undergoing therapy. Generally however, dosage will approximate that which is typical for known methods of administration of the specific pharmaceutically active agent. Persons of ordinary skill in the art can easily determine optimum dosages, dosing methodologies and repetition rates. The exact formulation and dosage can be chosen by the individual physician in view of the patient's condition (see, Fingl, et al., "The Pharmacological Basis of Therapeutics", Ch. 1 p. 1 (1975)).

Thus, depending on the severity and responsiveness of the condition to be treated, dosing can be a single or repetitive administration, with course of treatment lasting from several days to several weeks or until cure is effected or diminution of the disorder is achieved. In various embodiments an ophthalmic composition of the present invention may be administered from about 1 to 10 times daily or as needed to effectuate amelioration of a particular disorder or prevent wound progression. Further, the composition may be administered continuously or intermittently daily, weekly, monthly or yearly as needed.

In embodiments in which the ophthalmic composition includes a polypeptide PKC isoform inhibitor or activator, the polypeptide is provided in the composition at a concentration of between 0.001 and 100 µg/ml. For example, the concentration may be between 0.001 and 100, 0.01 and 50, 0.01 and 10, 0.01 and 1, and 0.01 and .5 µg/ml.

In one dosing protocol, the method comprises administering a peptide PKC isoform inhibitor or activator to the ophthalmic tissue of the subject, for example as a drop. The peptide is topically applied at a concentration of from about 1 µg/ml to about 1000 µg/ml, 1 µg/ml to about 500 µg/ml, 1µg/ml to about 100 µg/ml, 1 µg/ml to about 10 µg/ml, or 10 µg/ml to about 100 µg/ml. The peptide may be administered at least once daily, weekly, biweekly, or monthly until the condition is treated. For example, about 0.1 to 1.0 µg of peptide is administered per eye at least about 1, 2, 3, 4, 5, 6, 7 or 8 times per day.

In embodiments in which the ophthalmic composition includes insulin, insulin is provided in the composition at a concentration of between 0.001 and 100 µg/ml. For example, the concentration may be between 0.001 and 100, 0.01 and 50, 0.01 and 10, 0.01 and 1, and 0.01 and .5 µg/ml.

The following examples are provided to further illustrate the embodiments of the present disclosure, but are not intended to limit the scope. While they are typical of those that might be used, other procedures, methodologies, or techniques known to those skilled in the art may alternatively be used.

### EXAMPLE 1

### THERAPEUTIC EFFECT OF OPHTHALMIC BUFFER

Formula 1 was formulated for eye treatment and to enhance MPDY-1 mechanism of action. To determine the therapeutic effect of ophthalmic buffer Formula 1 on corneal ulcers and eye conditions, a series of experiments were performed utilizing rabbit eyes. Corneal ulcers were induced in the eyes of rabbits by either mechanical or chemical means and subsequently treated with Formula 1 buffer solution or Control. Corneal ulcers were induced as follows.

Mechanical Corneal Ulcer Formation: A 6mm corneal trephine (Grieshaber, Switzerland), preset for 50 micrometer in depth and a miniblade were used under a surgical microscope, to perform a uniform central corneal epithelial erosions, 6 mm in diameter and 50 micrometer in depth.

Chemical Alkaly Ulcer Formation: A 5mm absorption paper disk was used to perform corneal alkaly erosion by installation of 120 µl of NaOH IN for 10 seconds. Eyes were washed thoroughly with sterile irrigation water until pH returned to normal (∼pH=7).

Buffer compositions are shown in Table 2 (Sterodex® not shown).

**Table 2: Ophthalmology Buffer Compositions**

| Buffer Composition (w/v) | DPBS^{-/-} | BSS | NHBSS | Formula 1 |
|---|---|---|---|---|
| sodium chloride | 0.8% | 0.64%, | 0.8% | 0.71% |
| potassium chloride | 0.2% | 0.075% | 0.04% | 0.083% |
| dibasic sodium phosphate | 1.15% | | 0.036% | |
| Potassium Phosphate Monobasic | 0.2% | | 00.64% | |
| sodium bicarbonate | | | | |
| calcium chloride dihydrate | | 0.048% | | |
| Magnesium chloride hexahydrate | | 0.03% | | |
| sodium acetate trihydrate | | 0.39% | | 0.39% |
| trisodium citrate dihydrate | | 0.17% | | 0.17% |
| pH | 7.5 | 7.5 | 7.5 | 7.2 |
| Osmolality [mOsm/Kg] | 300 | 300 | 274 | 300 |

The healing kinetics of mechanically induced corneal ulcers after treatment with ophthalmic buffer Formula 1 versus Control buffer (DPBS^{-/-}) was tested. Rabbits were subjected to mechanical corneal wounding following the protocol discussed above. Eyes were treated by two daily ocular instillations of treatments during 3 successive days. Fluorescein staining was used to measure the corneal erosion area at twelve hours intervals starting 24 hrs post wounding and up to 72 hrs. As shown in Figure 1, the results demonstrate that treatment with Formula 1 accelerates healing of corneal erosions.

In addition to the time to heal parameter which is routinely assessed in wound healing studies, several publications established the importance of a new parameter, 50% reduction in wound size to serve as a robust predictor of complete wound closure. This surrogate marker was suggested by the authors to serve as a pivotal clinical decision point to predict efficiency of wound healing care in diabetic foot ulcers (see, Sheehan et al., Diabetes Care 26(6):1879-82 (2003); and Armstrong et al., Diabetes Care 31:26-29, (2008)). Results were therefore analyzed as percentage of eyes with 50% closure or higher at 24 hr post wounding (Figure 2).

Formula 1 was also demonstrated to promote complete healing of cornea erosions (Figure 3).

In addition to mechanically induced corneal ulceration, the effects of the designated treatments on chemically induced corneal ulceration via alkali burns (as performed above) to rabbit eyes were examined. Clinical observation data are summarized in Table 3 and results shown in Figure 4. The results demonstrate that buffer Formula 1 provides superior healing versus Control. Further, the data summarized in the graphs clearly demonstrate the beneficial effect of Formula 1 of reducing eye inflammation and vascularization.

**Table 3: Clinical Observations**

| **Parameter** | **Treatment** | **Day 2** | **Day 3** |
|---|---|---|---|
| **Lid-edema** | **Formula 1** | **0.4** | **0** |
| | **Control** | **1** | **0** |
| **Conjunctive-redness** | **Formula 1** | **1.6** | **0.4** |
| | **Control** | **2** | **1** |
| **Conjunctive-edema** | **Formula 1** | **0.4** | **0** |
| | **Control** | **1** | **0** |
| **Cornea-edema** | **Formula 1** | **1.2** | **0.6** |
| | **Control** | **2** | **1** |
| **Iris-vascular** | **Formula 1** | **0** | **0** |
| | **Control** | **1** | **0** |
| **Secretion** | **Formula 1** | **1.2** | **0.6** |
| | **Control** | **2** | **1** |

Further experiments were performed to assess ocular turbidity and scarring after 7 days of treatment with ophthalmic buffer solution Formula 1, BSS, Sterodex® (standard of care) and Control (water) in rabbit eyes subjected to chemical corneal wounding.

In one experiment, rabbit eyes were exposed to 1M NaOH on a 5mm paper-disc for 10 sec. The disc was removed and eyes were washed with irrigation of water until pH was normalized (pH = 6.8 - 7.2). Eyes were then treated 3 times daily, by the application of 120µl of the various buffer solutions for 7 days. The scar area was measured by Pictzar® software. Score of turbidity was calculated per scar areas (final area/initial area) and given by medical criteria. Score of turbidity was calculated per unhealed scar areas. Figure 5 shows that the score of turbidity per scar area after 7 days of treatment is reduced in eyes treated with Formula 1 as compared to Sterodex® and ControL

In another experiment, rabbit eyes were exposed to 1M NaOH on a 5mm paper-disc for 10 sec. The disc was removed and eyes were washed with irrigation of water until pH was normalized (pH = 6.8 - 7.2). Eyes were then treated 3 times daily, by the application of 120µL of the various buffer solutions for 7 days. The scar area was measured by Pictzar® software. Figure 6 shows that scar area after 7 days of treatment is reduced in eyes treated with Formula 1 as compared to Sterodex® and ControL

In another experiment, rabbit eyes were exposed to 1M NaOH on a 5mm paper-disc for 10 sec. The disc was removed and eyes were washed with irrigation of water until pH was normalized (pH = 6.8 - 7.2). Eyes were treated 3 times daily, by the application of 120µL of the various buffer solutions for 7 days. The scar area was measured by Pictzar® software. Score of turbidity was calculated per scar areas (final area/initial area) and given by medical criteria. Score of turbidity (given by medical criteria), was calculated per unhealed scar areas. Figure 7 shows that the score of turbidity per scar area after 7 days of treatment is reduced in eyes treated with Formula 1 as compared to BSS, Sterodex® and ControL

### EXAMPLE 2

### THERAPEUTIC EFFECT OF MPDY-1 ALONE AND IN COMBINATION WITH ADDITIONAL PHARMACEUTICAL ACTIVES

This example discusses experiments showing the therapeutic effect of MPDY-1 in BSS or in DPBS^{-/-} (referred to as Formula 2) and MPDY-1 with insulin in DPBS^{-/-} (referred to as Formula 3) or MPDY-1 in HOB-10 (referred to as HO/05/09) on mechanical and chemical corneal wounding.

Rabbits were subjected to mechanical corneal wounding as discussed in Example 1. Eyes were treated by two daily ocular instillations of treatments during 3 successive days. Fluorescein staining was used to measure the corneal erosion area at six hours intervals. Figure 8 shows a comparison of the percentage of eyes with full closure after treatment with vehicle alone (DPBS^{-/-}, left), Formula 3 (MPDY-1 0.1µg/eye/treatment and insulin 0.01µg /eye/treatment, (center), and Formula 2 (MPDY-1 0.1µg /eye/treatment, right). Formula 3 and Formula 2 clearly exhibit accelerated healing of corneal ulcers and eye injury.

Additional experiments were conducted using varying dosages of active agent. Rabbits were subjected to mechanical corneal wounding as described above. Eyes were treated by two daily ocular instillations of treatments during 3 successive days with 0.1 or 0.5µg/eye/treatment of MPDY-1 alone in HOB-10 (referred to as HO/05/09) or in BSS or in combination with insulin0.1-0.5µg/eye/treatment. Rabbits received a total of daily dose from 0.1µg (1µg/ml) and up to 2µg (20µg/ml). Fluorescein staining was used to measure the corneal erosion area at twelve hours intervals starting 24 hrs post wounding and up to 72 hr.

As shown in Figure 9, the results demonstrate that eyes treated with MPDY-1(in BSS) or HO/05/09 exhibited faster healing of ocular injury demonstrating closure of 60% as early as 24h post wounding versus Control animals that were treated with a standard of care ocular buffer. Moreover, HO/05/09 treated animals reached full closure as early as 48 hr post wounding.

As discussed previously, in addition to the time to heal parameter which is routinely assessed in wound healing studies, several publications established the importance of a new parameter, 50% reduction in wound size to serve as a robust predictor of complete wound closure. Results were therefore analyzed as percentage of eyes with 50% closure or higher at 24 hr post wounding (see Figure 10). The results clearly demonstrate that while none of the control eyes reached at least 50% closure of corneal ulcers, MPDY-1 in BSS (MPDY-1) or in Formula 1 (HO/05/09) achieved closure of 60% or higher of corneal ulcers.

Complete scar-less healing is vital for regaining eye site after injury. Therefore the ability of MPDY-1 alone and in combination with insulin to achieve complete corneal healing was investigated.

Rabbits were subjected to mechanical corneal wounding as described above. Eyes were treated by two daily ocular instillations of treatments during 3 successive days with 0.1 or 0.5µg/eye/treatment of MPDY-1 in BSS (MPDY-1) or in Formula 1 (HO/05/09) 0.1-0.5µg/eye/treatment. Rabbits received a total of daily dose from 0.1µg (1µg/ml) and up to 2µg (20µg/ml). Fluorescein staining was used to measure 100% healing of the corneal erosion area at 48, 60, and 72 hrs post wounding.

As evidenced in Figure 11, the results show that while only 30% of eyes in the control group treated with standard ophthalmic buffer (BSS) reached full scar-less closure within 72 hr post wounding treated eyes were completely healed without ocular scarring as early as 48 hr post wounding (30% and 65% MPDY-1 and HO/05/09 respectively). Furthermore, all eyes treated with HO/05/09 reached full closure within 60 hr and eyes treated with MPDY-1 within 72 hr post wounding.

In addition to mechanically induced corneal ulceration, the effects of the designated treatments on chemically induced corneal ulceration via alkali burns to rabbit eyes were examined. Clinical observation data are summarized in Table 4 and results shown in Figure 12. The results demonstrate that the treatment with MPDY-1 alone in HOB-10 (HO/05/09) and in combination with insulin provide superior healing versus Control. Further, the data summarized in the graphs clearly show that HO/05/09 and HO/05/09 + Insulin inhibit vascularization in the eye, and dramatically reduce eye inflammation.

**Table 4: Clinical Observations**

| **Parameter** | Treatment | Day 2 | Day 3 |
|---|---|---|---|
| Lid-edema | Control | 1 | 0 |
| | HO/05/09 5µg | 0 | 0 |
| | HO/05/09 5µg+Insulin | 0.2 | 0 |
| Conjunctive-redness | Control | 2 | 1 |
| | HO/05/09 5µg | 1.4 | 0.2 |
| | HO/05/09 5µg+Insulin | 1 | 0 |
| Conjunctive-edema | Control | 1 | 0 |
| | HO/05/09 5µg | 0.2 | 0 |
| | HO/05/09 5µg+Insulin | 0.4 | 0 |
| Cornea-edema | Control | 2 | 1 |
| | HO/05/09 5µg | 0.8 | 0 |
| | HO/05/09 5µg+Insulin | 0.6 | 0 |
| Iris-vascular | Control | 1 | 0 |
| | HO/05/09 5µg | 0 | 0 |
| | HO/05/09 5µg+Insulin | 0 | 0 |
| Secretion | Control | 2 | 1 |
| | HO/05/09 5µg | 0.4 | 0 |
| | HO/05/09 5µg+Insulin | 0.2 | 0 |

Further experiments were performed to assess ocular turbidity and scarring after 7 days of treatment with HO/05/09 with or without insulin, with Sterodex® (standard of care) and Control (water) in rabbit eyes subjected to chemical corneal wounding. Results are shown in Figure 13.

In one experiment, rabbit eyes were exposed to 1M NaOH on a 5mm paper-disc for 10 sec. The disc was removed and eyes were washed with irrigation of water until pH was normalized (pH = 6.8 - 7.2). Eyes were then treated 3 times daily, by the application of 120µL solution for 7 days. The eyes were treated with HO/05/09 (0.5µg/eye/treatment, 1.5 µg/eye/day), HO/05/09 + Insulin (0.5µg/eye/treatment, 1.5 µg/eye/day), Sterodex® (standard of care) and Control (water). Figure 14 shows that the score of turbidity per scar area after 7 days of treatment is reduced in eyes treated with HO/05/09 and HO/05/09 + Insulin as compared to Sterodex® and Control.

In another experiment, rabbit eyes were exposed to 1M NaOH on a 5mm paper-disc for 10 sec. The disc was removed and eyes were washed with irrigation of water until pH was normalized (pH = 6.8 - 7.2). Eyes were then treated 3 times daily, by the application of 120µL solution for 7 days. The eyes were treated with HO/05/09 (0.5µg/eye/treatment, 1.5µg/eye/day), HO/05/09 + Insulin (0.5µg/eye/treatment, 1.5 µg/eye/day), Sterodex® (standard of care) and Control (water). The scar area was measured by Pictzar® software. Figure 15 shows that the scar area after 7 days of treatment is reduced in eyes treated with HO/05/09 and HO/05/09 + Insulin as compared to Sterodex® and ControL

In another experiment, rabbit eyes were exposed to 1M NaOH on a 5mm paper-disc for 10 sec. The disc was removed and eyes were washed with irrigation of water until pH was normalized (pH = 6.8 - 7.2). Eyes were treated 3 times daily, by the application of 120µL solution for 7 days. The eyes were treated with HO/05/09 (0.5µg/eye/treatment, 1.5µg/eye/day), HO/05/09 + Insulin (0.5µg/eye/treatment, 1.5 µg/eye/day), Sterodex® (standard of care) and Control (water). The scar area was measured by Pictzar® software. Score of turbidity (given by medical criteria), was calculated per unhealed scar areas. Figure 16 shows that the score of turbidity per scar area after 7 days of treatment is reduced in eyes treated with HO/05/09 and HO/05/09 + Insulin as compared to Sterodex® and ControL

### EXAMPLE 3

### ANTI-INFLAMMATORY EFFECT OF MPDY-1

This example discusses experiments showing the anti-inflammatory effect of MPDY-1. The anti-inflammatory effect of MPDY-1 was demonstrated using various *in-vitro, ex-vivo* and *in-vivo* models. MPDY-1 was shown to attenuate ICAM-1 expression on endothelial cells and keratinocytes, inhibit macrophages, neutrophiles and T-cells infiltration into inflammation site and attenuate activation of macrophages at inflammation site.

### EXAMPLE 4

### THERAPEUTIC EFFECT OF MPDY-1

A series of experiments were performed to asses that therapeutic effect of MPDY-1 in various buffers. Buffer solutions were developed for MPDY-1 to fit both for eye treatment and for MPDY-1 mechanism of action. MPDY-1 at different concentrations was tested in two buffers that were developed (referred to as Formula 1 and NHBSS) as well as in a standard buffer for eye treatment (BSS) and DPBS^{-/-} which is typically used for skin wound therapy.

The various buffer compositions formulated are shown in Table 2 of Example 1.

The buffer solutions were formulated with and without MPDY-1 and tested for therapeutic effect using rabbit eyes having been subjected to mechanical corneal wounding as described in Example 1. Eyes were treated by two daily ocular instillations of treatments during 3 successive days. Fluorescein staining was used to measure the corneal erosion area at twelve hour intervals starting 24 hrs post wounding.

Results are shown in Figures 17-19. Formula 1 and Formula 1 including MPDY-1 (HO/05/09) are shown to accelerate wound closure as compared with other buffers and buffer/MPDY-1 combinations. Thus Formula 1 enhanced the mechanism of action of MPDY-1.

### EXAMPLE 5

### THERAPEUTIC EFFECT OF MPDY-1

Following the demonstration that HO/05/09 accelerates normal acute corneal wound healing (mechanical wounded), its ability to overcome wound healing impairments in a chronic wound healing model was examined. For this purpose, the drug was examined in a chemical erosion model. Alkali burning of corneas (6mm wide) was performed on anaesthetized rabbits using NaOH (IN) for 20 seconds. The eyes were irrigated for 30 seconds with 10ml sterile saline. Immediately after irrigation, eyes were stained with fluorescein and photographed. Eyes were treated for 7 days with HO/05/09 1µg/ml and 5µg/ml, or Sterodex® and Oflox™ (steroids and antibiotics) as standard of care in the clinical practice or left untreated as control.

Morphological assessment of corneal epithelial wounds 24 hours post wounding exhibited increased re-epithelialization in HO/05/09 treated eyes in both concentrations, as compared to untreated eyes or standard of care controls. As 50% closure was shown to be an important predictor for complete wound healing, the corneal erosions were assessed for 50% closure as well as for complete healing (98% closure on day 7). As shown in Figure 20, a dose dependent tendency was observed at 24h post wounding. In the 1µg/ml treated group, 60% of the eyes exhibited 50% closure at this early stage while in the 5µg/ml treated group 80% of the wounds were 50% closed. This trend of HO/05/09 beneficial effect was also observed at 48 hours time point where in both the 1µg/ml and 5µg/ml treated groups, 60% of the wounds exhibited above 98% wound closure while no animals in the control groups reached closure at this time point.

These results were confirmed utilizing immunohistochemistry staining for Keratin 12 (K12) that stains basal epithelial cells followed by histological analysis of corneal re-epithelialization and assessment of the quality of the new epithelial layer of the cornea. The results shown in Figures 21A-C demonstrate that 7 days following wounding, both of HO/05/09 treatment groups exhibited increased (60% at the HO/05/09 5µg/ml and almost 70% at the HO/05/09 1µg/ml) corneal closure as assessed by H&E staining, while in the control group, untreated and Sterodex® + Oflox™ treated, only 10% of the corneas reached closure. Moreover, both HO/05/09 treatment groups exhibited qualitative healing as expressed by K12 staining (Figures 21A and C).

Qualitative healing is not associated only with epithelial closure but also with regeneration of the cornea matrix fiber tissue underneath. As demonstrated in Figure 22, collagen histochemical staining demonstrates that seven days post wounding the collagen deposition was normalized in treated wounds. Similar results to a lower extent where observed in the Sterodex® + Oflox™ treated group while in the untreated control none of the wounds exhibited matrix remodeling of the cornea.

Finally, inflammation of the cornea was investigated by examining several morphological inflammatory parameters during seven days of treatment post chemical ulcer wounding. As demonstrated in Figure 23, HO/05/09 reduced local inflammatory response as expressed by a decrease in corneal edema, conjunctival erythema, conjunctival edema and discharge.

In addition Figure 24 shows a summary of average scoring for 12 different clinical inflammatory parameters from a similar study. The results show that HO/05/09 1µg/ml reduced local inflammation similar to Sterodex® and steroids, which is a very efficient agent to reduce local inflammation of the cornea. However, as shown above, the use of steroids dramatically reduces re-epithelialization and ulcer healing, while HO/05/09 exerts an opposite effect of accelerated healing.

### EXAMPLE 6

### THERAPEUTIC EFFECT OF DIFFERENT PKC ISOFORM MODULATORS

In another experiment, Alkali burning of corneas was performed on anaesthetized mice using Silver Nitrate applicators (75% Silver nitrate, 25% Potassium nitrate, Grafco, GFCO Inc.) for 10 seconds. The eyes were irrigated immediately with 5ml sterile saline. Eyes were treated by three daily ocular instillations of treatments during 7 successive days with HO/05/09 (1µg/ml and 5µg/ml) or Formula 1 as control.

The results demonstrated that the treatment with HO/05/09 in both concentrations provided superior healing ability versus control (Formula 1) and dramatically reduced eye inflammation. Treatment with HO/05/09 was found to be beneficial for corneal re-epithelialization (Figure 25) and reduction in eye inflammation, as measured by varied parameters, such as Lymphocytes infiltration (Figure 26), I-cam-1 expression (Figure 27) and recruitment of T-cells (Figure 28). In addition, treatment with HO/05/09 reduced pathological angiogenesis in wound area (Figure 29).

In another experiment, Alkali burning of corneas was performed on anaesthetized mice using Silver Nitrate applicators (75% Silver nitrate, 25% Potassium nitrate, Grafco, GFCO Inc.) for 10 seconds. The eyes were irrigated immediately with 5ml sterile saline. Eyes were treated 3 times daily for 3 or 7 days with DAP-1 1µg/ml, DIP-1 1µg/ml and EPIP-2 1µg/ml or Formula 1 as control. Treatment with DAP-1 1µg/ml, DIP-1 1µg/ml and EPIP-2 1µg/ml exhibited wound healing enhancement compare to control.

The results demonstrated that the treatment with the peptides affected the ability of the cornea to heal. Treatment with all the examined peptides was found to be beneficial for corneal re-epithelialization, while DIP-1 1µg/ml was found to be more potent (Figure 30). All examined peptides exhibited the ability to reduce inflammatory response in wound area to some extent. DAP-1 and DIP-1 excelled in reducing inflammatory cells infiltration into the wound area (Figure 31), while DAP-1 and EPIP-2 were found to be more potent in decreasing specific neutrophils recruitment (Figure 32). In addition, treatment with all examined peptides reduced pathological angiogenesis in wound area (Figure 33). Furthermore, treatment with DIP-1 or EPIP-2 led to normalization in pathological corneal swelling (Figure 34).

Although the disclosure has been described with reference to the above example, it will be understood that modifications and variations are encompassed within the spirit and scope of the disclosure. Accordingly, the disclosure is limited only by the following claims.

Embodiments of the invention corresponding to the published claims of PCT/IL2011/000866 (WO 2012/063237) include:
1. An ophthalmic composition comprising:
   a) 0.1 to 2.0% (w/v) sodium chloride;
   b) 0.01 to .5% (w/v) potassium chloride;
   c) 0.01 to 1.0% (w/v) sodium acetate trihydrate;
   d) 0.01 to 1.0% (w/v) trisodium citrate dihydrate;
   e) water; and
   f) one or more pharmaceutically active agents.
2. The ophthalmic composition of embodiment 1, wherein the composition comprises 0.6 to 0.8% (w/v) sodium chloride.
3. The ophthalmic composition of embodiment 1, wherein the composition comprises 0.07 to 0.09% (w/v) potassium chloride.
4. The ophthalmic composition of embodiment 1, wherein the composition comprises 0.3 to 0.5% (w/v) sodium acetate trihydrate.
5. The ophthalmic composition of embodiment 1, wherein the composition comprises 0.1 to 0.3% (w/v) trisodium citrate dihydrate.
6. The ophthalmic composition of embodiment 1, wherein the composition has a pH of about 5.5 to 8.0 or about 6.8 to 7.6.
7. The ophthalmic composition of embodiment 6, wherein the composition has a pH of about 7.2.
8. The ophthalmic composition of embodiment 1, wherein the composition has an osmolality from 220 to 320 mOsm/kg.
9. The ophthalmic composition of embodiment 1, wherein the composition has an osmolality of about 300 mOsm/kg.
10. The ophthalmic composition of embodiment 1, wherein the composition has a viscosity of about 1 to 50,000 cps.
11. The ophthalmic composition of embodiment 1, wherein the pharmaceutically active agent is selected from the group consisting of: anesthetic, astringent, anti-hypertensive, anti-glaucoma, neuro-protective, anti-allergy, muco-secretagogue, angiostatic, anti-microbial, pain-relieving and anti-inflammatory agents.
12. The ophthalmic composition of embodiment 11, wherein the pharmaceutically active agent is selected from the group consisting of: polypeptide, oligonucleotide, hormone, chemical compound, or lipid.
13. The ophthalmic composition of embodiment 1, wherein the pharmaceutically active agent is a PKC-α inhibitor, a PKC-ε inhibitor, a PKC-δ inhibitor or a PKC-δ activator.
14. The ophthalmic composition of embodiment 13, wherein the inhibitor is a polypeptide.
15. The ophthalmic composition of embodiment 14, wherein the inhibitor is between 5 and 20 amino acids in length.
16. The ophthalmic composition of embodiment 15, wherein the polypeptide comprises an amino acid sequence selected from SEQ ID NOs: 1-6, 12, 14, 17 and physiologically acceptable salts thereof.
17. The ophthalmic composition of embodiment 16, wherein the polypeptide comprises an N-terminal modification, C-terminal modification, or combination thereof.
18. The ophthalmic composition of embodiment 17, wherein the polypeptide is N-acylated.
19. The ophthalmic composition of embodiment 18, wherein the polypeptide is N-myristoylated or N-palmitoylated.
20. The ophthalmic composition of embodiment 16, wherein the polypeptide is selected from SEQ ID NOs: 7-11, 13, 15, 16 and 18.
21. The ophthalmic composition of embodiment 1, wherein the pharmaceutically active agent is insulin.
22. The ophthalmic composition of embodiment 13, wherein the composition further comprises insulin.
23. The ophthalmic composition of embodiment 13, wherein the composition comprises insulin in combination with a PKC-α inhibitor.
24. The ophthalmic composition of embodiment 1, wherein the composition further comprises a buffering agent, preservative, tonicity agent, demulcent, wetting agent, surfactant, solubilizing agent, stabilizing agent, comfort enhancing agent, emollient, pH-adjusting agent, lubricant, aggregation inhibitory agent, charge modifying agent, degradative enzyme inhibitor, membrane penetration enhancer, sequestering agent (chelating agent), vasodilator or viscosity adjusting agent.
25. The ophthalmic composition of embodiment 1, wherein the composition includes less than 0.03% (w/v) of calcium chloride dihydrate or magnesium chloride hexahydrate.
26. An ophthalmic composition comprising:
   a) 0.1 to 2.0% (w/v) sodium chloride;
   b) 0.01 to .5% (w/v) potassium chloride;
   c) 0.01 to 1.0% (w/v) sodium acetate trihydrate;
   d) 0.01 to 1.0% (w/v) trisodium citrate dihydrate; and
   e) water, with the proviso that the composition includes less than 0.03% (w/v) of calcium chloride dihydrate or magnesium chloride hexahydrate.
27. The ophthalmic composition of embodiment 26, wherein the composition comprises 0.6 to 0.8% (w/v) sodium chloride.
28. The ophthalmic composition of embodiment 26, wherein the composition comprises 0.07 to 0.09% (w/v) potassium chloride.
29. The ophthalmic composition of embodiment 26, wherein the composition comprises 0.3 to 0.5% (w/v) sodium acetate trihydrate.
30. The ophthalmic composition of embodiment 26, wherein the composition comprises 0.1 to 0.3% (w/v) trisodium citrate dihydrate.
31. The ophthalmic composition of embodiment 26, wherein the composition has a pH of about 5.5 to 8.0 or about 6.8 to 7.6.
32. The ophthalmic composition of embodiment 31, wherein the composition has a pH of about 7.2.
33. The ophthalmic composition of embodiment 26, wherein the composition has an osmolality from 220 to 320 mOsm/kg.
34. The ophthalmic composition of embodiment 26, wherein the composition has an osmolality of about 300 mOsm/kg.
35. The ophthalmic composition of embodiment 26, wherein the composition has a viscosity of about 1 to 50,000 cps.
36. The ophthalmic composition of embodiment 26, wherein the composition further comprises one or more pharmaceutically active agents.
37. The ophthalmic composition of embodiment 36, wherein the pharmaceutically active agent is selected from the group consisting of: anesthetic, astringent, anti-hypertensive, anti-glaucoma, neuro-protective, anti-allergy, muco-secretagogue, angiostatic, anti-microbial, pain-relieving and anti-inflammatory agents.
38. The ophthalmic composition of embodiment 36 wherein the pharmaceutically active agent is selected from the group consisting of: polypeptide, oligonucleotide, hormone, chemical compound, or lipid.
39. The ophthalmic composition of embodiment 36, wherein the pharmaceutically active agent is a PKC-α inhibitor, a PKC-ε inhibitor, a PKC-δ inhibitor or a PKC-δ activator.
40. The ophthalmic composition of embodiment 39, wherein the inhibitor is a polypeptide.
41. The ophthalmic composition of embodiment 40, wherein the inhibitor is between 5 and 20 amino acids in length.
42. The ophthalmic composition of embodiment 41, wherein the polypeptide comprises an amino acid sequence selected from SEQ ID NOs: 1-6, 12, 14, 17 and physiologically acceptable salts thereof.
43. The ophthalmic composition of embodiment 42, wherein the polypeptide comprises an N-terminal modification, C-terminal modification, or combination thereof.
44. The ophthalmic composition of embodiment 43, wherein the polypeptide is N-acylated.
45. The ophthalmic composition of embodiment 44, wherein the polypeptide is N-myristoylated or N-palmitoylated.
46. The ophthalmic composition of embodiment 42, wherein the polypeptide is selected from SEQ ID NOs: 7-11, 13, 15, 16 and 18.
47. The ophthalmic composition of embodiment 36, wherein the pharmaceutically active agent is insulin.
48. The ophthalmic composition of embodiment 39, wherein the composition further comprises insulin.
49. The ophthalmic composition of embodiment 48, wherein the composition comprises insulin in combination with a PKC-α inhibitor.
50. The ophthalmic composition of embodiment 26, wherein the composition further comprises a buffering agent, preservative, tonicity agent, demulcent, wetting agent, surfactant, solubilizing agent, stabilizing agent, comfort enhancing agent, emollient, pH-adjusting agent, lubricant, aggregation inhibitory agent, charge modifying agent, degradative enzyme inhibitor, membrane penetration enhancer, sequestering agent (chelating agent), vasodilator or viscosity adjusting agent.
51. The ophthalmic composition of embodiment 26, wherein the composition is adapted for use as an ocular lubricant or artificial tear composition.
52. A method of accelerating or promoting healing of damaged ocular tissue or an ocular wound in a subject, comprising administering an ophthalmic composition to an eye of a subject, wherein the ophthalmic composition comprises:
   a) 0.1 to 2.0% (w/v) sodium chloride;
   b) 0.01 to .5% (w/v) potassium chloride;
   c) 0.01 to 1.0% (w/v) sodium acetate trihydrate;
   d) 0.01 to 1.0% (w/v) trisodium citrate dihydrate; and
   e) water.
53. The method of embodiment 52, wherein the composition comprises 0.6 to 0.8% (w/v) sodium chloride.
54. The method of embodiment 52, wherein the composition comprises 0.07 to 0.09% (w/v) potassium chloride.
55. The method of embodiment 52, wherein the composition comprises 0.3 to 0.5% (w/v) sodium acetate trihydrate.
56. The method of embodiment 52, wherein the composition comprises 0.1 to 0.3% (w/v) trisodium citrate dihydrate.
57. The method of embodiment 52, wherein the composition has a pH of about 5.5 to 8.0 or about 6.8 to 7.6.
58. The method of embodiment 57, wherein the composition has a pH of about 7.2.
59. The method of embodiment 52, wherein the composition has an osmolality from 220 to 320 mOsm/kg.
60. The method of embodiment 59, wherein the composition has an osmolality of about 300 mOsm/kg.
61. The method of embodiment 52, wherein the composition has a viscosity of about 1 to 50,000 cps.
62. The method of embodiment 52, wherein the composition further comprises a buffering agent, preservative, tonicity agent, demulcent, wetting agent, surfactant, solubilizing agent, stabilizing agent, comfort enhancing agent, emollient, pH-adjusting agent, lubricant, aggregation inhibitory agent, charge modifying agent, degradative enzyme inhibitor, membrane penetration enhancer, sequestering agent (chelating agent), vasodilator or viscosity adjusting agent.
63. The method of embodiment 52, wherein the composition further comprises one or more pharmaceutically active agents.
64. The method of embodiment 63, wherein the pharmaceutically active agent is selected from the group consisting of: anesthetic, astringent, anti-hypertensive, anti-glaucoma, neuro-protective, anti-allergy, muco-secretagogue, angiostatic, anti-microbial, pain-relieving and anti-inflammatory agents.
65. The method of embodiment 63, wherein the pharmaceutically active agent is selected from the group consisting of: polypeptide, oligonucleotide, hormone, chemical compound, or lipid.
66. The method of embodiment 65, wherein the pharmaceutically active agent is a polypeptide.
67. The method of embodiment 63, wherein the pharmaceutically active agent is a PKC-α inhibitor, a PKC-ε inhibitor, a PKC-δ inhibitor or a PKC-δ activator.
68. The method of embodiment 67, wherein the inhibitor is a polypeptide.
69. The method of embodiment 68, wherein the inhibitor is between 5 and 20 amino acids in length.
70. The method of embodiment 69, wherein the polypeptide comprises an amino acid sequence selected from SEQ ID NOs: 1-6, 12, 14, 17 and physiologically acceptable salts thereof.
71. The method of embodiment 70, wherein the polypeptide comprises an N-terminal modification, C-terminal modification, or combination thereof.
72. The method of embodiment 71, wherein the polypeptide is N-acylated.
73. The method of embodiment 72, wherein the polypeptide is N-myristoylated or N-palmitoylated.
74. The method of embodiment 70, wherein the polypeptide is selected from SEQ ID NOs: 7-11, 13, 15, 16 and 18.
75. The method of embodiment 67, wherein the composition further comprises insulin.
76. The method of embodiment 68, wherein the polypeptide is present in the composition at a concentration of between 0.001 and 100 µg/ml.
77. The method of embodiment 68, wherein the peptide is a PKC-α inhibitor.
78. The method of embodiment 77, wherein the composition further comprises insulin.
79. The method of embodiment 78, wherein the polypeptide and insulin are each present in the composition at a concentration of between 0.001 and 100 µg/ml.
80. The method of embodiment 52, wherein the composition is administered between 1 and 10 times per day.
81. The method of embodiment 69, wherein the composition is administered 3 times per day.
82. The method of embodiment 63, wherein the pharmaceutically active agent is insulin.
83. The method of embodiment 82, wherein the insulin is present in the composition at a concentration of between 0.001 and 100 µg/ml.
84. The method of embodiment 52, wherein the wound is selected from the group consisting of: a corneal ulceration wound, a retinopathy wound, a burn, an inflammation wound, a dry eye syndrome wound, a macular degeneration wound, a laceration, a surgical incision wound, or a post surgical adhesion wound.
85. The method of embodiment 52, wherein the composition includes less than 0.03% (w/v) of calcium chloride dihydrate or magnesium chloride hexahydrate.
86. A method of lubricating an eye comprising, topically administering an ophthalmic composition to an eye of a subject, wherein the ophthalmic composition comprises:
   a) 0.1 to 2.0% (w/v) sodium chloride;
   b) 0.01 to .5% (w/v) potassium chloride;
   c) 0.01 to 1.0% (w/v) sodium acetate trihydrate;
   d) 0.01 to 1.0% (w/v) trisodium citrate dihydrate; and
   e) water.
87. The method of embodiment 86, wherein the composition comprises 0.6 to 0.8% (w/v) sodium chloride.
88. The method of embodiment 86, wherein the composition comprises 0.07 to 0.09% (w/v) potassium chloride.
89. The method of embodiment 86, wherein the composition comprises 0.3 to 0.5% (w/v) sodium acetate trihydrate.
90. The method of embodiment 86, wherein the composition comprises 0.1 to 0.3% (w/v) trisodium citrate dihydrate.
91. The method of embodiment 86, wherein the composition has a pH of about 5.5 to 8.0 or about 6.8 to 7.6.
92. The method of embodiment 86, wherein the composition has a pH of about 7.2.
93. The method of embodiment 86, wherein the composition has an osmolality from 220 to 320 mOsm/kg.
94. The method of embodiment 93, wherein the composition has an osmolality of about 300 mOsm/kg.
95. The method of embodiment 86, wherein the composition has a viscosity of about 1 to 50,000 cps.
96. The method of embodiment 86, wherein the composition further comprises a buffering agent, preservative, tonicity agent, demulcent, wetting agent, surfactant, solubilizing agent, stabilizing agent, comfort enhancing agent, emollient, pH-adjusting agent, lubricant, aggregation inhibitory agent, charge modifying agent, degradative enzyme inhibitor, membrane penetration enhancer, sequestering agent (chelating agent), vasodilator or viscosity adjusting agent.
97. The method of embodiment 86, wherein the composition includes less than 0.03% (w/v) of calcium chloride dihydrate or magnesium chloride hexahydrate.
98. The method of embodiment 86, wherein the composition further comprises one or more pharmaceutically active agents.
99. The method of embodiment 98, wherein the pharmaceutically active agent is selected from the group consisting of: anesthetic, astringent, anti-hypertensive, anti-glaucoma, neuro-protective, anti-allergy, muco-secretagogue, angiostatic, anti-microbial, pain-relieving and anti-inflammatory agents.
100. The method of embodiment 98, wherein the pharmaceutically active agent is selected from the group consisting of: polypeptide, oligonucleotide, hormone, chemical compound, or lipid.
101. A method of delivering a pharmaceutical agent to a subject comprising, topically administering an ophthalmic composition to an eye of the subject, wherein the ophthalmic composition comprises:
   a) 0.1 to 2.0% (w/v) sodium chloride;
   b) 0.01 to .5% (w/v) potassium chloride;
   c) 0.01 to 1.0% (w/v) sodium acetate trihydrate;
   d) 0.01 to 1.0% (w/v) trisodium citrate dihydrate;
   e) water, and
   f) a pharmaceutically active agent.
102. The method of embodiment 101, wherein the composition comprises 0.6 to 0.8% (w/v) sodium chloride.
103. The method of embodiment 101, wherein the composition comprises 0.07 to 0.09% (w/v) potassium chloride.
104. The method of embodiment 101, wherein the composition comprises 0.3 to 0.5% (w/v) sodium acetate trihydrate.
105. The method of embodiment 101, wherein the composition comprises 0.1 to 0.3% (w/v) trisodium citrate dihydrate.
106. The method of embodiment 101, wherein the composition has a pH of about 5.5 to 8.0 or about 6.8 to 7.6.
107. The method of embodiment 101, wherein the composition has a pH of about 7.2.
108. The method of embodiment 101, wherein the composition has an osmolality from 220 to 320 mOsm/kg.
109. The method of embodiment 108, wherein the composition has an osmolality of about 300 mOsm/kg.
110. The method of embodiment 101, wherein the composition has a viscosity of about 1 to 50,000 cps.
111. The method of embodiment 101, wherein the pharmaceutically active agent is selected from the group consisting of: anesthetic, astringent, anti-hypertensive, anti-glaucoma, neuro-protective, anti-allergy, muco-secretagogue, angiostatic, anti-microbial, pain-relieving and anti-inflammatory agents.
112. The method of embodiment 101, wherein the pharmaceutically active agent is selected from the group consisting of: peptide, oligonucleotide, hormone, chemical compound, or lipid.
113. The method of embodiment 112, wherein the pharmaceutically active agent is a polypeptide.
114. The method of embodiment 101, wherein the pharmaceutically active agent is a PKC-α inhibitor, a PKC-ε inhibitor, a PKC-δ inhibitor or a PKC-δ activator.
115. The method of embodiment 114, wherein the inhibitor is a polypeptide.
116. The method of embodiment 115, wherein the inhibitor is between 5 and 20 amino acids in length.
117. The method of embodiment 116, wherein the polypeptide comprises an amino acid sequence selected from SEQ ID NOs: 1-6, 12, 14, 17 and physiologically acceptable salts thereof.
118. The method of embodiment 117, wherein the polypeptide comprises an N-terminal modification, C-terminal modification, or combination thereof.
119. The method of embodiment 118, wherein the polypeptide is N-acylated.
120. The method of embodiment 119, wherein the polypeptide is N-myristoylated or N-palmitoylated.
121. The method of embodiment 117, wherein the polypeptide is selected from SEQ ID NOs: 7-11, 13, 15, 16 and 18.
122. The method of embodiment 114, wherein the composition further comprises insulin.
123. The method of embodiment 115, wherein the polypeptide is present in the composition at a concentration of between 0.001 and 100 µg/ml.
124. The method of embodiment 113, wherein the peptide is a PKC-α inhibitor.
125. The method of embodiment 124, wherein the composition further comprises insulin.
126. The method of embodiment 125, wherein the polypeptide and insulin are each present in the composition at a concentration of between 0.001 and 100 µg/ml.
127. The method of embodiment 101, wherein the composition is administered between 1 and 10 times per day.
128. The method of embodiment 127, wherein the composition is administered 3 times per day.
129. The method of embodiment 101, wherein the pharmaceutically active agent is insulin.
130. The method of embodiment 129, wherein the insulin is present in the composition at a concentration of between 0.001 and 100 µg/ml.
131. The method of embodiment 101, wherein the composition further comprises a buffering agent, preservative, tonicity agent, demulcent, wetting agent, surfactant, solubilizing agent, stabilizing agent, comfort enhancing agent, emollient, pH-adjusting agent, lubricant, aggregation inhibitory agent, charge modifying agent, degradative enzyme inhibitor, membrane penetration enhancer, sequestering agent (chelating agent), vasodilator or viscosity adjusting agent.
132. The method of embodiment 101, wherein the composition includes less than 0.03% (w/v) of calcium chloride dihydrate or magnesium chloride hexahydrate.
133. A kit comprising the ophthalmic composition of embodiments 1 or 26.
134. The kit of embodiment 133, wherein the kit further comprises instructions for administering the composition.

## Claims

1. Use of a PKC-α inhibitor or a physiologically acceptable salt thereof in the preparation of an ophthalmic composition for treating a dry eye in a subject.

2. The use of claim 1, wherein the composition further comprises a buffering agent, preservative, pharmaceutically active agent, tonicity agent, demulcent, wetting agent, surfactant, solubilizing agent, stabilizing agent, comfort enhancing agent, emollient, pH-adjusting agent, lubricant, aggregation inhibitory agent, charge modifying agent, degradative enzyme inhibitor, membrane penetration enhancer, sequestering agent (chelating agent), vasodilator or a viscosity adjusting agent, and wherein the pharmaceutically active agent is selected from the group consisting of steroids, anesthetic, astringent, anti-hypertensive, anti-glaucoma, neuro-protective, anti-allergy, muco-secretagogue, angiostatic, anti-microbial, pain-relieving and anti-inflammatory agents.

3. The use of claim 1, wherein the PKC-α inhibitor is a polypeptide comprising an amino acid sequence selected from SEQ ID NOs: 1-11.

4. The use of claim 3, wherein the polypeptide is N-acylated, N-myristoylated or N-palmitoylated.

5. The use of claim 3, wherein the composition further comprises insulin.

6. The use of claim 5, wherein the polypeptide and insulin are each present in the composition at a concentration of between 0.001 and 100 µg/ml.

7. The use of claim 1, wherein the composition is administered between 1 and 10 times per day.

8. The use of claim 1, wherein the composition is effective in treating eye inflammation or an inflammatory condition of the eye.

9. Use of a PKC-α inhibitor or a physiologically acceptable salt thereof in the preparation of an ophthalmic composition for lubricating an eye in a subject.

10. The use of claim 9, wherein the composition further comprises a buffering agent, preservative, pharmaceutically active agent, tonicity agent, demulcent, wetting agent, surfactant, solubilizing agent, stabilizing agent, comfort enhancing agent, emollient, pH-adjusting agent, lubricant, aggregation inhibitory agent, charge modifying agent, degradative enzyme inhibitor, membrane penetration enhancer, sequestering agent (chelating agent), vasodilator or a viscosity adjusting agent, and wherein the pharmaceutically active agent is selected from the group consisting of steroids, anesthetic, astringent, anti-hypertensive, anti-glaucoma, neuro-protective, anti-allergy, muco-secretagogue, angiostatic, anti-microbial, pain-relieving and anti-inflammatory agents.

11. The use of claim 9, wherein the PKC-α inhibitor is a polypeptide comprising an amino acid sequence selected from SEQ ID NOs: 1-11.

12. The use of claim 11, wherein the polypeptide is N-acylated, N-myristoylated or N-palmitoylated.

13. The use of claim 11, wherein the composition further comprises insulin.

14. The use of claim 13, wherein the polypeptide and insulin are each present in the composition at a concentration of between 0.001 and 100 µg/ml.

15. The use of claim 10, wherein the composition is administered between 1 and 10 times per day.


SEQUENCE LISTING

<110>  Arava Bio-Tech Ltd.

<120>  BUFFERED OPHTHALMIC COMPOSITIONS AND USE THEREOF

<130>  P-78295-EP1

<150>  EP 11840613.1
<151>  2011-11-08

<150>  PCT/IL2011/000866
<151>  2011-11-08

<150>  US 61/411,464
<151>  2010-11-08

<150>  US 61/411,466
<151>  2010-11-08

<160>  18

<170>  PatentIn version 3.5

<210>  1
<211>  6
<212>  PRT
<213>  Artificial Sequence

<220>
<223>  Synthetic construct

<400>  1

Phe Ala Arg Lys Gly Ala
1               5


<210>  2
<211>  9
<212>  PRT
<213>  Artificial Sequence

<220>
<223>  Synthetic construct

<400>  2

Phe Ala Arg Lys Gly Ala Leu Arg Gln
1               5


<210>  3
<211>  9
<212>  PRT
<213>  Artificial Sequence

<220>
<223>  Synthetic construct

<400>  3

Phe Ala Arg Lys Gly Ala Leu Arg Gln

1                 5


<210>  4
<211>  7
<212>  PRT
<213>  Artificial Sequence

<220>
<223>  Synthetic construct

<400>  4

Phe Ala Arg Lys Gly Ala Leu
1               5


<210>  5
<211>  8
<212>  PRT
<213>  Artificial Sequence

<220>
<223>  Synthetic construct

<400>  5

Phe Ala Arg Lys Gly Ala Arg Gln
1               5


<210>  6
<211>  8
<212>  PRT
<213>  Artificial Sequence

<220>
<223>  Synthetic construct

<400>  6

Thr Leu Asn Pro Gln Trp Glu Ser
1               5


<210>  7
<211>  9
<212>  PRT
<213>  Artificial Sequence

<220>
<223>  Synthetic construct


<220>
<221>  MOD_RES
<222>  (1)..(1)
<223>  ACETYLATION addition of Myristoyl

<220>
<221>  MOD_RES
<222>  (6)..(6)
<223>  Alanine-13C3-15N

<220>
<221>  MISC_FEATURE
<222>  (9)..(9)
<223>  C terminal Hydroxyl

<400>  7

Phe Ala Arg Lys Gly Ala Leu Arg Gln
1               5


<210>  8
<211>  9
<212>  PRT
<213>  Artificial Sequence

<220>
<223>  Synthetic construct


<220>
<221>  MISC_FEATURE
<222>  (1)..(1)
<223>  N terminal Hydrogen

<220>
<221>  MISC_FEATURE
<222>  (9)..(9)
<223>  C terminal Hydroxyl

<400>  8

Phe Ala Arg Lys Gly Ala Leu Arg Gln
1               5


<210>  9
<211>  7
<212>  PRT
<213>  Artificial Sequence

<220>
<223>  Synthetic construct


<220>
<221>  MOD_RES
<222>  (1)..(1)
<223>  ACETYLATION addition of Myristoyl

<220>
<221>  MOD_RES
<222>  (7)..(7)
<223>  C terminal addition of Hydroxyl group and trifluoracetate salt

<400>  9

Phe Ala Arg Lys Gly Ala Leu
1               5


<210>  10

<211>  8
<212>  PRT
<213>  Artificial Sequence

<220>
<223>  Synthetic construct



<220>
<221>  MISC_FEATURE
<222>  (1)..(1)
<223>  N terminal Hydrogen

<220>
<221>  MISC_FEATURE
<222>  (8)..(8)
<223>  C terminal Hydroxyl

<400>  10

Thr Leu Asn Pro Gln Trp Glu Ser
1               5



<210>  11
<211>  8
<212>  PRT
<213>  Artificial Sequence

<220>
<223>  Synthetic construct



<220>
<221>  MOD_RES
<222>  (1)..(1)
<223>  ACETYLATION addition of Palmitoyl

<220>
<221>  MOD_RES
<222>  (8)..(8)
<223>  C terminal Hydroxyl

<400>  11

Phe Ala Arg Lys Gly Ala Arg Gln
1               5



<210>  12
<211>  8
<212>  PRT
<213>  Artificial Sequence

<220>
<223>  Synthetic construct

<400>  12

Pro Tyr Ile Ala Leu Asn Val Asp
1               5

<210>  13
<211>  8
<212>  PRT
<213>  Artificial Sequence

<220>
<223>  Synthetic construct


<220>
<221>  MISC_FEATURE
<222>  (1)..(1)
<223>  N terminal Hydrogen

<220>
<221>  MISC_FEATURE
<222>  (8)..(8)
<223>  C terminal Hydroxyl

<400>  13

Pro Tyr Ile Ala Leu Asn Val Asp
1               5


<210>  14
<211>  10
<212>  PRT
<213>  Artificial Sequence

<220>
<223>  Synthetic construct

<400>  14

Ser Phe Asn Ser Tyr Glu Leu Gly Ser Leu
1               5                   10


<210>  15
<211>  10
<212>  PRT
<213>  Artificial Sequence

<220>
<223>  Synthetic construct


<220>
<221>  MISC_FEATURE
<222>  (10)..(10)
<223>  C terminal Hydroxyl

<400>  15

Ser Phe Asn Ser Tyr Glu Leu Gly Ser Leu
1               5                   10


<210>  16
<211>  10
<212>  PRT
<213>  Artificial Sequence

<220>
<223>  Synthetic construct


<220>
<221>  MOD_RES
<222>  (1)..(1)
<223>  ACETYLATION addition of Myristoyl

<220>
<221>  MISC_FEATURE
<222>  (10)..(10)
<223>  C terminal Hydroxyl

<400>  16

Ser Phe Asn Ser Tyr Glu Leu Gly Ser Leu
1               5                   10


<210>  17
<211>  12
<212>  PRT
<213>  Artificial Sequence

<220>
<223>  Synthetic construct

<400>  17

Met Arg Ala Ala Glu Ala Ala Ala Ala Glu Pro Met
1               5                   10


<210>  18
<211>  12
<212>  PRT
<213>  Artificial Sequence

<220>
<223>  Synthetic construct


<220>
<221>  MISC_FEATURE
<222>  (1)..(1)
<223>  N terminal Hydrogen

<220>
<221>  MISC_FEATURE
<222>  (12)..(12)
<223>  C terminal Hydroxyl

<400>  18

Met Arg Ala Ala Glu Ala Ala Ala Ala Glu Pro Met
1               5                   10